# EUROPEAN PATENT APPLICATION

(11) **EP 3 552 573 A1**
(43) Date of publication of application: **16.10.2019**
(21) Application number: 19160703.5
(22) Date of filing: 05.03.2019
(51) Int. Cl.: A61B 34/30, A61B 34/35, A61B 90/60, A61B 34/00, B25J 19/02, A61B 90/00, A61B 17/00

(54) **REMOTE CONTROL APPARATUS AND METHOD OF IDENTIFYING TARGET PEDAL**

(30) Priority: 13.04.2018 JP 2018077383; 30.11.2018 JP 2018224677
(71) Applicant: Medicaroid Corporation, Kobe 650-0047 (JP)
(72) Inventor: Ishihara, Kazuki, Kobe-shi, Hyogo 650-0047 (JP); Horita, Shiro, Kobe-shi, Hyogo 651-0083 (JP); Nagase, Syuuichi, Yokohama-shi, Kanagawa 220-6218 (JP)
(74) Representative: Hoffmann Eitle

(57) **Abstract**

A remote control apparatus includes an operating handle, an operation pedal unit (2), and a control unit. The operating handle is for remotely operating medical equipment. The operation pedal unit (2) includes a plurality of pedals (20) each of which is operated when pressed downward by a foot to perform a function related to the medical equipment, and a sensor (26) detecting the presence of the foot operating the pedals (20). The control unit receives detection information from the sensor (26). When the control unit receives detection information from a plurality of sensors (26) arranged near the pedal (20), the control unit determines that the foot operating the pedal (20) is present.

## Description

### TECHNICAL FIELD

The present invention relates to a remote control apparatus, a surgical system, and a method of identifying a target pedal.

### BACKGROUND ART

A remote control apparatus for operating medical equipment has been known.

Japanese Translation of PCT Application No. 2015-534476 (Patent Document 1) discloses a remote control apparatus provided with a foot unit including a plurality of foot pedals, and presence sensors detecting the presence of a foot of an operator.

The remote control apparatus of Patent Document 1 is configured to detect the presence of the operator's foot, and to highlight, on a display device of the remote control apparatus, an icon of a surgical instrument linked to the corresponding foot pedal to inform the operator of the foot pedal being operated. According to Patent Document 1, the remote control apparatus is configured to detect the presence of the operator's foot near one or more foot pedals, using a presence sensor.

### SUMMARY

The remote control apparatus of Patent Document 1 detects the presence of the operator's foot near one or more foot pedals using the presence sensor. Therefore, in a situation where the presence sensor detects the foot near a plurality of foot pedals, it is sometimes difficult to identify a single target pedal among the plurality of pedals. This may disadvantageously decrease the accuracy in detecting the presence of the foot which operates the target pedal among the plurality of pedals.

The present invention intends to provide a remote control apparatus which can reduce the decrease of the accuracy in detecting the presence of the foot which operates the target pedal among the plurality of pedals, and a surgical system.

A first aspect of the invention may be directed to a remote control apparatus including an operating handle, an operation pedal unit, and a control unit. The operating handle may be for remotely operating medical equipment. The operation pedal unit may include a plurality of pedals each of which is operated when pressed downward by a foot to perform a function related to the medical equipment, and a sensor detecting the presence of the foot operating the pedals. The control unit may receive detection information from the sensor. The sensor may include a plurality of sensors arranged near a first pedal among the plurality of pedals. When the control unit receives the detection information from the plurality of sensors arranged near the first pedal, the control unit may determine that the foot operating the first pedal is present.

The "foot" to be detected is not limited to a bare foot of the operator, and may also include a shoe or sock put (or worn) on the operator's foot.

A second aspect of the invention may be directed to a method for identifying a target pedal to be operated among a plurality of pedals each of which is operated when pressed downward by a foot to perform a function related to medical equipment. The method for identifying a target pedal to be operated may include: determining whether a plurality of sensors arranged near any one of the pedals has detected the foot operating the pedal; and identifying, if the plurality of sensors has detected the presence of the foot operating the pedal, the pedal near the plurality of sensors as the target pedal.

The present invention can reduce the decrease of the accuracy in detecting the presence of the foot which operates a target pedal among a plurality of pedals.

### BRIEF DESCRIPTION OF THE DRAWINGS

FIG. 1 is a diagram schematically illustrating a remote control apparatus according to a first embodiment.
FIG. 2 is a diagram illustrating the remote control apparatus according to the first embodiment to which a scope type display unit is attached.
FIG. 3 is a block diagram illustrating a control configuration of the remote control apparatus according to the first embodiment.
FIG. 4 is a perspective view illustrating an operation pedal unit of the remote control apparatus according to the first embodiment.
FIG. 5 is a plan view illustrating the operation pedal unit of the remote control apparatus according to the first embodiment.
FIG. 6 is a front view illustrating the operation pedal unit of the remote control apparatus according to the first embodiment.
FIG. 7 is a diagram illustrating an example of allocation of the operation pedal unit of the remote control apparatus according to the first embodiment.
FIG. 8 is a side view illustrating the remote control apparatus according to the first embodiment in a first posture.
FIG. 9 is a side view illustrating the remote control apparatus according to the first embodiment in a second posture.
FIG. 10 is a diagram illustrating a model of an operator of the remote control apparatus according to the first embodiment.
FIG. 11 is a diagram illustrating the remote control apparatus according to the first embodiment to which a non-scope type display unit is attached.
FIG. 12 is a schematic diagram illustrating a first example of a lock mechanism and an unlock mechanism of the remote control apparatus according to the first embodiment.
FIG. 13 is a schematic diagram illustrating a second example of a lock mechanism and an unlock mechanism of the remote control apparatus according to the first embodiment.
FIG. 14 is a schematic diagram illustrating a third example of a lock mechanism and an unlock mechanism of the remote control apparatus according to the first embodiment.
FIG. 15 is a flowchart for explaining a target pedal identification process performed by a control unit of the remote control apparatus according to the first embodiment.
FIG. 16 is a diagram illustrating a remote control apparatus according to a second embodiment.
FIG. 17 is a diagram illustrating a remote control apparatus according to a third embodiment.
FIG. 18 is a diagram illustrating a remote control apparatus according to a fourth embodiment.
FIG. 19 is a diagram illustrating a variation of the remote control apparatus according to the first to fourth embodiments.

### DETAILED DESCRIPTION

One or more embodiments will be described below with reference to the drawings.

### [First Embodiment]

### (Configuration of Remote Control Apparatus)

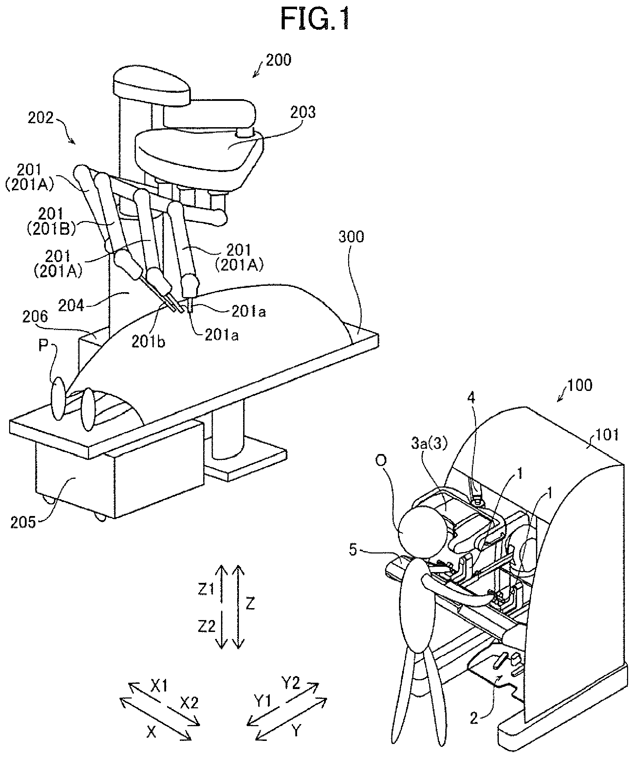

A configuration of a remote control apparatus 100 according to a first embodiment will be described with reference to FIGS. 1 to 14.

As shown in FIG. 1, the remote control apparatus 100 is used to remotely operate medical equipment provided for a patient-side system 200. When an operator O, who is a surgeon, enters a movement type instruction to be executed by the patient-side system 200 to the remote control apparatus 100, the remote control apparatus 100 transmits the movement type instruction to the patient-side system 200 via a controller 206. The patient-side system 200 then handles the medical equipment, such as a surgical instrument or an endoscope gripped by a surgical manipulator 201, in response to the movement type instruction transmitted from the remote control apparatus 100. A minimally invasive operation is performed in this manner. A surgical system includes the remote control apparatus 100, and the patient-side system 200 having the surgical manipulator 201. The patient-side system 200 is an example of a "patient-side apparatus" recited in the claims. The endoscope 201b is an example of an "imaging unit" recited in the claims.

The patient-side system 200 may constitute an interface through which a surgery is performed on a patient P. The patient-side system 200 is arranged beside a surgical table 300 on which the patient P lies. The patient-side system 200 includes a plurality of surgical manipulators 201, one of which grips the endoscope 201b, and the other surgical manipulators 201 grip surgical instruments (instruments 201a). The surgical manipulators 201 each gripping the surgical instrument (the instrument 201a) function as instrument arms 201A. The surgical manipulator 201 gripping the endoscope 201b functions as a camera arm 201B. The instrument arms 201A and the camera arm 201B are supported by the same platform 203. Each of the plurality of surgical manipulators 201 has a plurality of joints. A driving section including a servomotor and a position detector such as an encoder are provided for each joint. Each surgical manipulator 201 is configured to be controlled such that the medical equipment attached to the surgical manipulator 201 makes desired movement in response to a driving signal given via the controller 206.

The platform 203 is supported by a positioner 202 placed on the floor of the operating room. The positioner 202 includes a column 204 having a lifting shaft adjustable in a vertical direction, and the column 204 is coupled to a base 205 having wheels and movable on the floor surface.

The instrument arm 201A detachably holds the instrument 201a as the medical equipment at a distal end thereof. The instrument 201a includes a housing attached to the instrument arm 201A, and an end effector attached to a distal end of an elongate shaft. Examples of the end effector include, but are not limited to, a grasping forceps, scissors, a hook, a high-frequency knife, a snare wire, a clamp, and a stapler, and may include various types of treatment tools. In a surgery using the patient-side system 200, each of the instrument arms 201A may be introduced into the body of the patient P through a cannula (a trocar) retained on the body surface of the patient P, so that the end effector of the instrument 201a is positioned close to a surgical site.

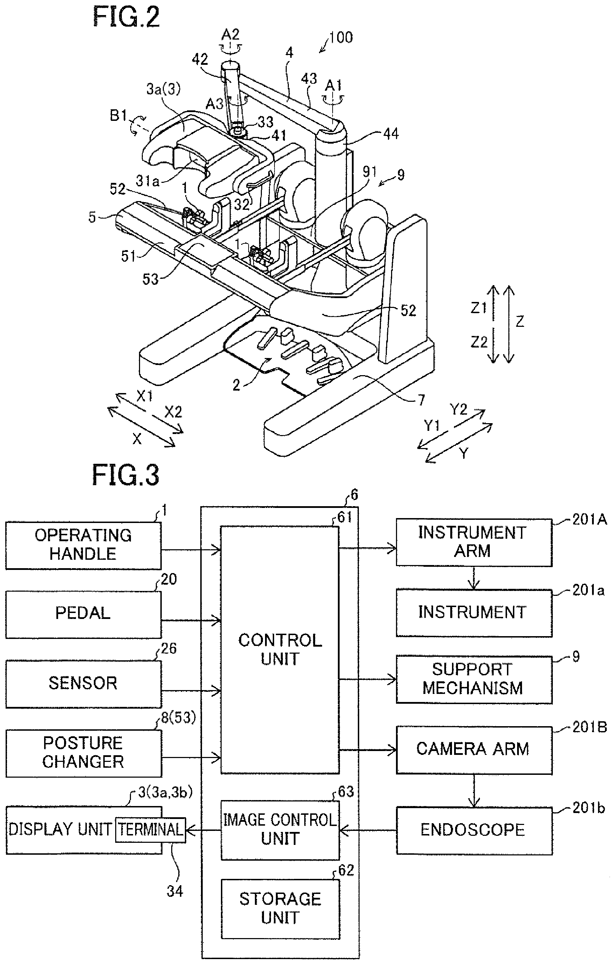

The endoscope 201b (see FIG. 3) as the medical equipment is detachably attached to the distal end of the camera arm 201B. The endoscope 201b takes images in the body cavity of the patient P. The images taken are output to the remote control apparatus 100. Examples of the endoscope 201b include a 3D endoscope capable of taking three-dimensional images, and a 2D endoscope. In a surgery using the patient-side system 200, the camera arm 201B is introduced into the body of the patient P through a trocar retained on the body surface of the patient P, so that the endoscope 201b is positioned close to the surgical site.

The remote control apparatus 100 constitutes an interface with the operator O. The remote control apparatus 100 serves as a device through which the operator O operates the medical equipment gripped by each surgical manipulator 201. That is, the remote control apparatus 100 is configured to be able to transmit, to the patient-side system 200, the movement type instruction that has been input by the operator O and that should be executed by the instrument 201a and the endoscope 201b, via the controller 206. The remote control apparatus 100 is installed beside the surgical table 300 so that the operator can check the condition of the patient P while operating the master, for example. The remote control apparatus 100 may be configured, for example, to wirelessly transmit the movement type instruction, and may be installed in a room different from the operating room where the surgical table 300 is placed.

The term "movement type" to be performed by the instrument 201a may refer to the type of movement (a series of positions and orientations) of the instrument 201a and the type of movement executed by the function of the respective instruments 201a. For example, if the instrument 201a is a grasping forceps, the movement type to be performed by the instrument 201a may include positions of rolling and pitching of a wrist of the end effector, and opening and closing the jaws. If the instrument 201a is a high-frequency knife, the movement type to be executed by the instrument 201a may include vibration of the high-frequency knife, specifically, a current supply to the high-frequency knife. If the instrument 201a is a snare wire, the movement type to be performed by the instrument 201a may include tightening, and releasing from the tightening. In addition, the movement type may include a movement of burning off a target site of the surgery, using a bipolar or a monopolar to which an electric current is supplied.

Examples of the movement type to be performed by the endoscope 201b include positioning, and determination of the orientation, of the tip end of the endoscope 201b, or setting of zoom magnification of the endoscope 201b.

The remote control apparatus 100 is provided with a cover 101 as shown in FIG. 1. The cover 101 is provided to cover left and right surfaces (facing X directions), a back surface (facing a Y2 direction), and a top surface (facing a Z1 direction), of the remote control apparatus 100. FIG. 2 and other subsequent drawings show the remote control apparatus 100 with its cover 101 removed for convenience sake.

As shown in FIGS. 2 and 3, the remote control apparatus 100 includes an operating handle 1, an operation pedal unit 2, a display unit supporting arm 4 supporting a display unit 3, an arm rest 5 which supports the arms of the operator O, a control device 6, and a base 7. The remote control apparatus 100 further includes a posture changer 8, and a support mechanism 9 which supports the operating handle 1 and the arm rest 5.

The operating handle 1 is provided to remotely operate the medical equipment gripped by each of the surgical manipulators 201. Specifically, the operating handle 1 accepts the operation conducted by the operator O to control the medical equipment (i.e., the instrument 201a and the endoscope 201b). The operating handle 1 includes a pair of operating handles 1 arranged in the X direction. Specifically, one of the pair of operating handles 1 toward an X2 direction (the right side) is operated by the right hand of the operator O, and the other one of the pair of operating handles 1 toward an X1 direction (the left side) is operated by the left hand of the operator O.

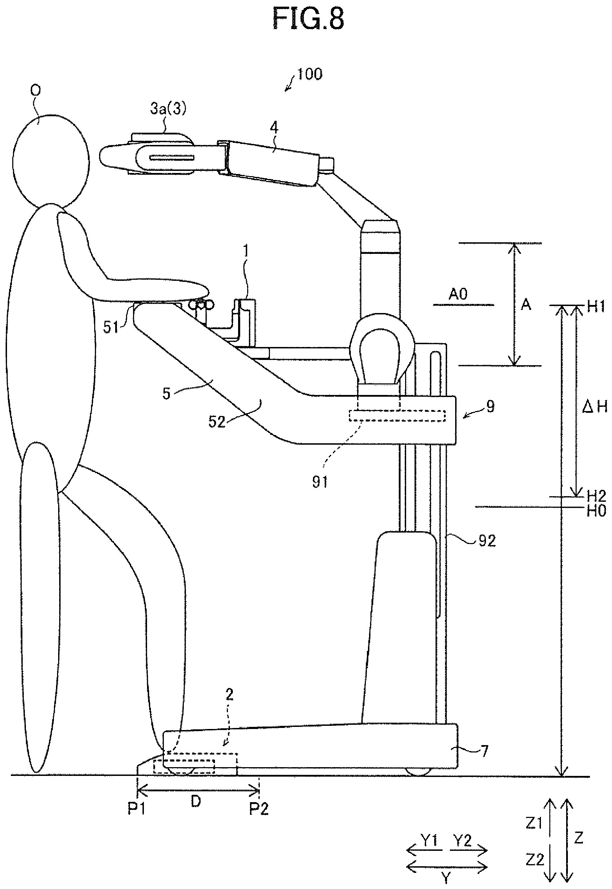

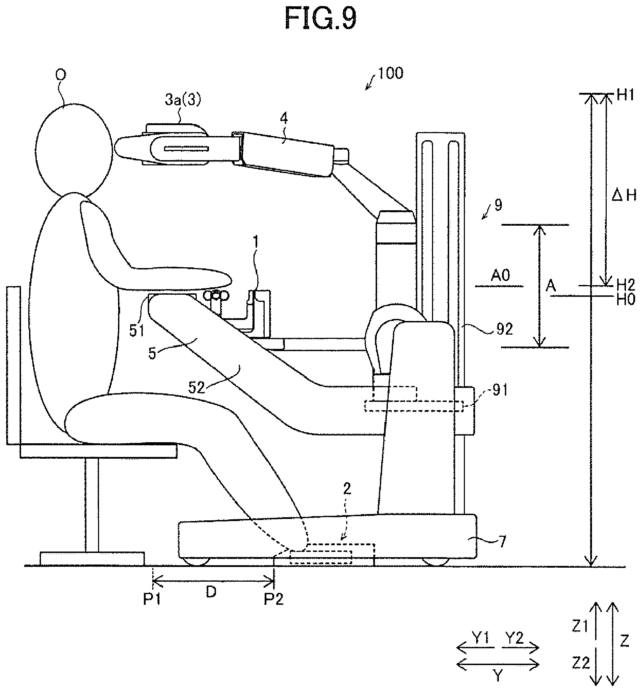

The operating handle 1 is attached to a support 91 of the support mechanism 9. The operating handle 1 is arranged to extend from the back side (Y2 direction) toward the front side (Y1 direction) of the remote control apparatus 100. A plurality of joints are provided between the support 91 and the operating handle 1. The operating handle 1 is configured to be movable within a predetermined three-dimensional operation area A (see FIGS. 8 and 9) with respect to the support 91. Specifically, the operating handle 1 is configured to be movable up and down (Z directions), the leftward and rightward directions (X directions), and the forward and backward directions (Y directions), with respect to the support 91. Each of the joints between the support 91 and the operating handle 1 is provided with a position detector (not shown), which detects a positional relationship between the respective joints. The position detector may be, for example, an encoder, a resolver, or a potentiometer, which may be used to detect a position of the operating handle 1 relative to the support 91.

The remote control apparatus 100 and the patient-side system 200 constitute a master-slave system in controlling the movements of the instrument arm 201A and the camera arm 201B. Specifically, the operating handle 1 serves as a master controlling element in the master-slave system, and the instrument arms 201A and the camera arm 201B gripping the medical equipment serve as slave moving elements. When the operator O operates the operating handle 1, the movement of the instrument arms 201A or the camera arm 201B is controlled so that the distal end of each instrument arm 201A (i.e., the end effector of the instrument 201a) or the distal end of the camera arm 201B (i.e., the endoscope 201b) will trace the movement of the operating handle 1 and shift accordingly.

The patient-side system 200 is configured to control the movements of the respective instrument arms 201A according to a movement scale factor which has been set. For example, in a case in which the movement scale factor has been set to be 1/2, the end effector of the instrument 201a is controlled to shift by one half (1/2) of a distance by which the operating handle 1 has shifted, which enables a fine level of accuracy for a surgery. The operating handle 1 is attached to the base 7, and extends in the Y direction toward the operator O.

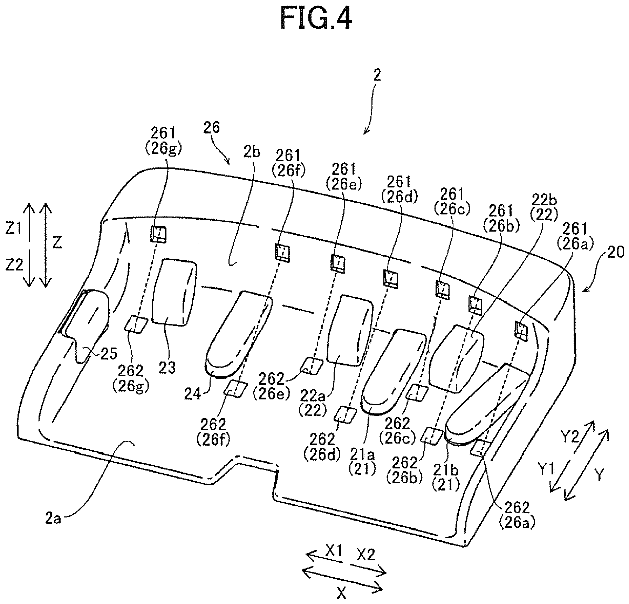

The operation pedal unit 2 includes a plurality of pedals 20 for executing the function related to the medical equipment, as shown in FIGS. 4 to 6. The operation pedal unit 2 includes a base portion 2a on which the plurality of pedals 20 are arranged, and a wall portion 2b standing upright from the base portion 2a. The pedals 20 include a coagulation pedal 21, a cutting pedal 22, a camera pedal 23, and a clutch pedal 24. The operation pedal unit 2 also includes a side pedal 25. The coagulation pedal 21, the cutting pedal 22, the camera pedal 23, and the clutch pedal 24 are operated when pressed downward by a foot. The side pedal 25 is operated when pressed in the horizontal direction. The pedals 20 accept the operation when pressed by about 10 mm, for example. The pedals 20 are configured such that they are operated with a pressing force as small as possible in accordance with the standard. The pedals 20 are operated by the foot of the operator O. The coagulation pedal 21 and the cutting pedal 22 are examples of a "first pedal" recited in the claims. The camera pedal 23 and the clutch pedal 24 are examples of a "second pedal" recited in the claims.

The operation pedal unit 2 includes a plurality of sensors 26 detecting the presence of the foot operating the pedals 20. The sensors 26 include sensors 26a, 26b, 26c, 26d, 26e, 26f, and 26g. Each of the plurality of sensors 26 includes a light emitter 261 and a light receiver 262. The sensor 26 detects the presence of the foot when the foot blocks light from the light emitter 261 to interrupt the reception of the light by the light receiver 262. That is, the sensor 26 is a blocking sensor. One of the light emitter 261 and the light receiver 262 is provided at the base portion 2a. The other one of the light emitter 261 and the light receiver 262 is provided at the wall portion 2b.

Detection information of the sensor 26 is transmitted to a control unit 61. The control unit 61, which has received the detection information, determines whether the foot operating the pedal 20 corresponding to the sensor 26 is present or not.

The coagulation pedal 21 enables the operation of coagulating the surgical site using a surgical instrument. Specifically, when the coagulation pedal 21 is operated, a voltage for coagulation is applied to the instrument 201a, thereby coagulating the surgical site. The coagulation pedal 21 includes coagulation pedals 21a and 21b. That is, a plurality of (two) coagulation pedals 21 are provided. The coagulation pedal 21a is located on the left (toward the X1 direction) of the coagulation pedal 21b. The coagulation pedal 21a is used, for example, in association with the instrument 201a of the instrument arm 201A operated via the left operating handle 1. The coagulation pedal 21b is used, for example, in association with the instrument 201 a of the instrument arm 201A operated via the right operating handle 1.

The cutting pedal 22 enables the operation of cutting the surgical site using a surgical instrument. Specifically, when the cutting pedal 22 is operated, a voltage for the cutting is applied to the instrument 201a, thereby cutting the surgical site. The cutting pedal 22 includes cutting pedals 22a and 22b. That is, a plurality of (two) cutting pedals 22 are provided. The cutting pedal 22a is located on the left (toward the X1 direction) of the cutting pedal 22b. The cutting pedal 22a is used, for example, in association with the instrument 201a of the instrument arm 201A operated via the left operating handle 1. The cutting pedal 22b is used, for example, in association with the instrument 201a of the instrument arm 201A operated via the right operating handle 1.

The camera pedal 23 is used to change the position and orientation of the endoscope 201b taking images of the body cavity. Specifically, the camera pedal 23 validates the operation of the endoscope 201b through the operating handle 1. That is, while the camera pedal 23 is pressed down, the position and orientation of the endoscope 201b can be changed by using the operating handle 1. For example, the endoscope 201b is operated using both of the left and right operating handles 1. Specifically, the endoscope 201b rotates when the right and left operating handles 1 rotate about a midpoint between the left and right operating handles 1. The endoscope 201b moves forward when the left and right operating handles 1 are pushed backward. The endoscope 201b moves backward when the left and right operating handles 1 are pulled forward. The endoscope 201b moves up and down, and leftward and rightward when the left and right operating handles 1 are moved up and down, and leftward and rightward.

The clutch pedal 24 is used to temporarily block (disconnect) the connection for operation between the surgical manipulator 201 and the operating handle 1 to stop the movement of the surgical instrument. Specifically, while the clutch pedal 24 is pressed down, the surgical manipulator 201 of the patient-side system 200 does not move even if the operating handle 1 is moved. For example, when the operating handle 1 comes close to the end of the movable range thereof, the clutch pedal 24 is pressed down to temporarily block the connection for operation so that the operating handle 1 can be returned to the center position. When the clutch pedal 24 is no longer pressed down, the surgical manipulator 201 and the operating handle 1 are connected again, and the operating handle 1 can be operated again at around the center position.

The side pedal 25 is used for the switching among the instrument arms 201A controlled by the operating handle 1. For example, there are four surgical manipulators 201, and two instrument arms 201A to be operated by the left and right operating handles 1 are selected from the three instrument arms 201A, except for the camera arm 201B, through the operation of the side pedal 25. The side pedal 25 is operated when pressed to the left (toward the X1 direction). For example, the instrument arm 201A to be operated by the right operating handle 1 is changed through the operation of the side pedal 25. That is, the instrument arm 201A operated by the left operating handle 1 is not changed, whereas the instrument arm 201A to be operated by the right operating handle 1 is changed.

As shown in FIGS. 4 to 6, the pedals 20, namely, the side pedal 25, the camera pedal 23, the clutch pedal 24, the cutting pedal 22a, the coagulation pedal 21a, the cutting pedal 22b, and the coagulation pedal 21b, are arranged in this order from left (X1) to right (X2). Further, the plurality of pedals 20 (the camera pedal 23, the clutch pedal 24, the cutting pedal 22a, the coagulation pedal 21a, the cutting pedal 22b, and the coagulation pedal 21b) are arranged in a width direction (X direction) of the operation pedal unit 2.

Specifically, the coagulation pedals 21 and the cutting pedals 22 are alternately arranged in the horizontal direction (the width direction (X direction) of the operation pedal unit 2). Thus, each of the plurality of coagulation pedals 21 and the plurality of cutting pedals 22 is operated in association with a corresponding one of the instruments 201a operated by the left and right operating handles 1. Further, the plurality of coagulation pedals 21 and the plurality of cutting pedals 22 are alternately and sequentially arranged on one side (the right side) of the operation pedal unit 2 with respect to the center of the operation pedal unit 2 in the width direction (X direction).

The arrangement of the pedals shown in FIGS. 4 to 6 is suitable for a case where only the right foot of the operator operates sets of the coagulation pedal 21 and the cutting pedal 22 respectively allocated to the right and left operating handles 1. If a set of the coagulation pedal 21a and the cutting pedal 22a is arranged on the left (toward the X1 direction) of the camera pedal 23 and the clutch pedal 24, and the camera pedal 23 and the clutch pedal 24 are arranged at the center (between the set of the coagulation pedal 21a and the cutting pedal 22a and the set of the coagulation pedal 21b and the cutting pedal 22b), the coagulation pedal 21a and the cutting pedal 22a allocated to the left operating handle 1 are suitably operated by the left foot, whereas the coagulation pedal 21b and the cutting pedal 22b allocated to the right operating handle 1 are suitably operated by the right foot.

On the operation pedal unit 2, the plurality of pedals 20 operated when pressed downward are arranged such that their positions in the planar direction do not overlap with each other, and that their vertical positions overlap with each other. Specifically, the plurality of pedals 20 are arranged such that their positions differ in the width direction (X direction), and that at least portions of the pedals 20 are located at the same vertical position. This configuration does not require the foot to greatly move up and down, unlike a case where the plurality of pedals 20 are arranged at two different vertical positions. That is, the plurality of pedals 20 including the coagulation pedals 21 and the cutting pedals 22 can be operated by the toe of the foot, while the heel of the foot is kept in contact with the floor. This can improve the ease of operation of the pedals 20 while maintaining the number of types of operations to be input.

More specifically, the coagulation pedals 21, the cutting pedals 22, the camera pedal 23, and the clutch pedal 24 are arranged such that their positions in the planar direction do not overlap with each other, and that their vertical positions overlap with each other.

In a preferred embodiment, the side pedal 25 is also arranged such that it can be operated by the foot of the operator with the heel kept in contact with the floor. Further, the side pedal 25 is preferably arranged not to overlap in the planar direction with the plurality of pedals to be pressed downward, and to overlap in the vertical direction with the pedals to be pressed downward. The side pedal 25 may be changed to a pedal operated when pressed downward.

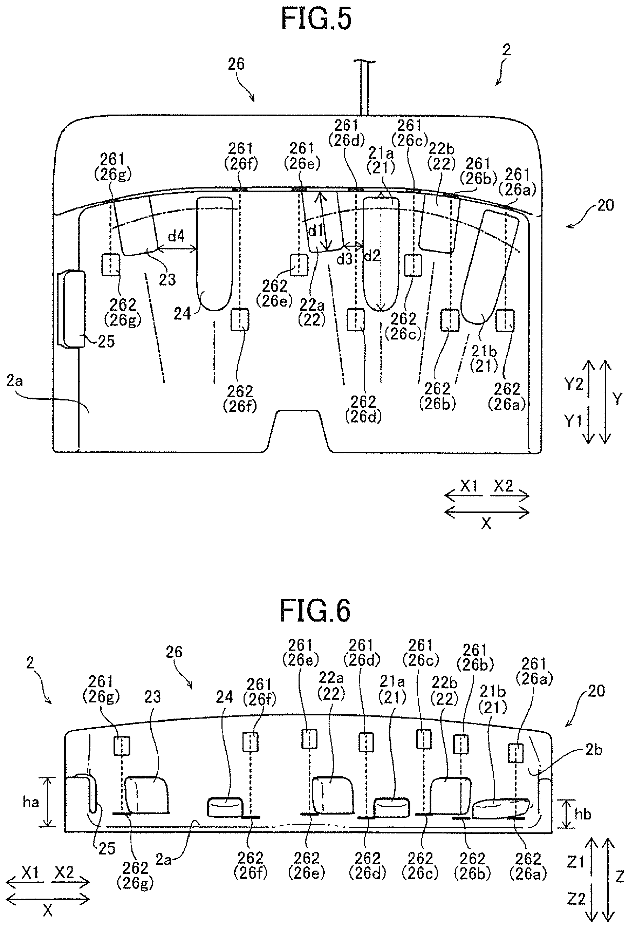

Among the plurality of pedals 20 to be pressed downward, pedals 20 adjacent to each other have top ends located at different vertical positions. Thus, the operator can distinguish the type of the pedal 20 without visually checking the pedal 20, and therefore, can operate the pedal 20 while looking at the display unit 3. As shown in FIG. 6, for example, top ends of the cutting pedals 22 and the camera pedal 23 are located at a vertical position away from the floor surface by a distance ha. Top ends of the coagulation pedals 21 and the clutch pedal 24 are located at a vertical position away from the floor surface by a distance hb.

Specifically, the vertical position of the top ends of the cutting pedals 22 is a first vertical position, and the vertical position of the top ends of the coagulation pedals 21 is a second vertical position different from the first vertical position. This configuration can reduce the risk of erroneous operation of the cutting pedals 22 and the coagulation pedals 21. Note that the top ends of the cutting pedals 22 may be at the second vertical position, and the top ends of the coagulation pedals 21 may be at the first vertical position.

The distance ha of the first vertical position from the floor surface is preferably 1.5 times or more the distance hb of the second vertical position from the floor surface. This configuration allows the operator to easily distinguish the cutting pedals 22 and the coagulation pedals 21 from one another without visually checking them. The distance ha of the first vertical position from the floor surface is about twice the distance hb of the second vertical position from the floor surface. This configuration allows the operator to distinguish the cutting pedals 22 and the coagulation pedals 21 from one another more easily, and can prevent the first vertical position to be too high.

The distance ha of the first vertical position from the floor surface is, for example, about 50 mm. The distance hb of the second vertical position from the floor surface is, for example, about 25 mm. This configuration allows the operator O to operate each of the cutting pedals 22 and the coagulation pedals 21 with the heel kept in contact with the floor. Thus, the operator O does not have to greatly lift the foot up. The distance ha of the first vertical position from the floor surface may be in a range of about 10 mm or more and about 200 mm or less. The distance hb of the second vertical position from the floor surface may be in a range of about 5 mm or more and about 100 mm or less.

As shown in FIG. 6, the plurality of pedals 20 of the operation pedal unit 2 are arranged such that their bottom ends are located substantially at the same vertical position.

The positions of the top and bottom ends of the side pedal 25 are arbitrarily determined. However, since the larger contact area of the pedal is more preferable, the top end of the side pedal 25 is suitably located at a higher vertical position, and the bottom end of the side pedal 25 is suitably located at a lower vertical position. In the example shown in FIG. 6, the top and bottom ends of the side pedal 25 are located at the same vertical positions as the top and bottom ends of the camera pedal 23.

As shown in FIG. 5, for example, the cutting pedals 22 and the camera pedal 23 protrude by a distance d1 when viewed in plan (when viewed in the Z direction). The coagulation pedals 21 and the clutch pedal 24 protrude by a distance d2 when viewed in plan. For example, the distance d2 is greater than the distance d1. This configuration allows the operator to easily distinguish between types of the pedals 20 and operate intrested pedals 20. When viewed in plan, the protruding distances of the pedals 20 may be different from each other, or may be substantially the same. In this case, the pedals 20 may protrude by the same distance in a fan-shaped pattern.

The coagulation pedals 21 and the clutch pedal 24 are different in shape when viewed in plan. This configuration, too, allows the operator to easily distinguish between the types of pedals 20 and operate the interested pedals 20. Specifically, the pedals 20 different in shape are alternately arranged in the horizontal direction.

As shown in FIG. 5, the operation pedal unit 2 includes the plurality of pedals 20 arranged in a fan-shaped pattern when viewed in plan (when viewed in the Z direction). Specifically, when viewed in plan, some of the plurality of pedals 20 are arranged in a fan-shaped pattern on one side of the operation pedal unit 2 with respect to the center thereof, and the other pedals 20 are arranged in a fan-shaped pattern on the other side of the operation pedal unit 2 with respect to the center. With the plurality of pedals 20 arranged in the fan-shaped pattern with respect to the operator O at the center, the operator O can operate the plurality of pedals 20 by pivoting the foot on the heel, kept in contact with the floor, and thereby moving the toe. This can improve the ease of operation of the pedals 20 while maintaining the number of types of operations to be input.

As shown in FIGS. 4 and 5, for example, the camera pedal 23 and the clutch pedal 24 are arranged on the left side part (toward the X1 direction) of the operation pedal unit 2 with respect to the center thereof. The camera pedal 23 is arranged to incline toward the right (the X2 direction) as it extends forward (the Y1 direction). The clutch pedal 24 is arranged to extend substantially in the forward and backward directions (the Y directions). This configuration allows the operator O to easily operate the camera pedal 23 and the clutch pedal 24 with the left foot.

The coagulation pedals 21 (21a and 21b) and the cutting pedals 22 (22a and 22b) are arranged on the right side part (toward the X2 direction) of the operation pedal unit 2 with respect to the center thereof. The cutting pedal 22a is arranged to incline toward the right (the X2 direction) as it extends forward (the Y1 direction). The coagulation pedal 21a is arranged to extend substantially in the forward and backward directions (the Y directions). The cutting pedal 22b is arranged to incline toward the left (the X1 direction) as it extends forward (the Y1 direction). The coagulation pedal 21b is arranged to incline further toward the left (the X1 direction) as it extends forward (the Y1 direction). This configuration allows the operator O to easily operate the coagulation pedals 21 (21a and 21b) and the cutting pedals 22 (22a and 22b) with the right foot.

The base portion 2a on which the plurality of pedals 20 are arranged can move in the horizontal direction. Specifically, both ends of the base portion 2a in the width direction (the X direction) are coupled to the base 7 of the remote control apparatus 100 via slide bearings, such that the operation pedal unit 2 can slidably move in a depth direction (the forward and backward directions, or the Y directions). The operation pedal unit 2 can be electrically moved in the depth direction by a driving device, such as a motor, provided in the base 7 of the remote control apparatus 100. This configuration can adjust the position of the pedals 20 according to the posture, physique, or preference of the operator O.

According to the first embodiment, the number of sensors 26 detecting the presence of the foot is more than the number of pedals 20 to be pressed downward. Specifically, seven sensors 26 are provided. The pedals 20 operated when pressed downward include six pedals (i.e., the coagulation pedals 21a and 21b, the cutting pedals 22a and 22b, the camera pedal 23, and the clutch pedal 24). This means that there are five sensors that detect the presence of the foot operating the two coagulation pedals 21 and the two cutting pedals 22.

According to the first embodiment, a plurality of sensors 26 are provided near a particular one of the plurality of pedals 20 in order to detect the foot operating the particular pedal 20. Using these sensors 26, it is determined whether the foot operating or is about to operate the particular pedal 20 is present or not. Specifically, receiving detection information from the plurality of sensors 26 arranged near a particular one of the plurality of pedals 20, the control unit 61 determines that the foot operating the particular pedal 20 is present. More specifically, receiving detection information from the plurality of sensors 26 arranged near the pedal 20 (the cutting pedal 22a, the coagulation pedal 21a, the cutting pedal 22b, the coagulation pedal 21 a), the control unit 61 determines that the foot operating the pedal 20 (the cutting pedal 22a, the coagulation pedal 21a, the cutting pedal 22b, the coagulation pedal 21a) is present. Further, for the determination of the presence of the foot operating a particular one (a first pedal, i.e., the coagulation pedal 21 and the cutting pedal 22) of the pedals 20, and the determination of the presence of the foot operating the pedal 20 adjacent to the particular pedal 20 (a first pedal, i.e., the coagulation pedal 21 and the cutting pedal 22), the control unit 61 uses the detection information sent from the sensor 26 arranged between the particular pedal 20 and the pedal 20 adjacent to the particular pedal 20. Specifically, the control unit 61 uses the detection information sent from the sensor 26b arranged between the coagulation pedal 21b and the cutting pedal 22b in order to determine whether the foot operating the coagulation pedal 21b is present or not, and whether the foot operating the cutting pedal 22b adjacent to the coagulation pedal 21b is present or not. Likewise, the control unit 61 uses the detection information sent from the sensors 26c arranged between the cutting pedal 22b and the coagulation pedal 21a in order to determine whether the foot operating the cutting pedal 22b is present or not, and whether the foot operating the coagulation pedal 21a adjacent to the cutting pedal 22b is present or not. Further, the control unit 61 uses the detection information sent from the sensor 26d arranged between the coagulation pedal 21a and the cutting pedal 22a in order to determine whether the foot operating the coagulation pedal 21a is present or not, and whether the foot operating the cutting pedal 22a adjacent to the coagulation pedal 21 a is present or not.

In this way, using two or more sensors 26, the presence of the foot approaching a target pedal 20 (the first pedal, i.e., the coagulation pedal 21 and the cutting pedal 22) can be detected. Thus, the pedal 20 to which the foot of the operator O makes an approach can be identified with accuracy. This configuration can improve the accuracy in detecting the presence of the foot operating the target pedal 20 among the plurality of pedals 20.

According to the first embodiment, at least one sensor 26 is provided near each of the plurality of pedals 20 in order to detect the foot operating the pedal 20. Thus, the foot approaching each pedal 20 can be detected. Further, a single sensor 26 is shared among a plurality of pedals 20 (the first pedals, i.e., the coagulation pedal 21 and the cutting pedal 22)) in order to detect the foot operating the pedals 20. That is, at least one of the plurality of sensors 26 is arranged between a particular one of the pedals 20 (the first pedal, i.e., the coagulation pedal 21 and the cutting pedal 22) and a pedal 20 adjacent to the particular pedal 20. Specifically, a particular one of the sensors 26 arranged between the center of a tip end (an end toward the Y1 direction) of a particular pedal 20 and the center of a tip end (an end toward the Y1 direction) of a pedal 20 adjacent to the particular pedal 20 is used to detect the presence of the foot operating each of the pedals 20 on the sides of the sensor (if the sensor is a light emitting-receiving sensor, linear light emitted from the light emitter is used). This configuration can prevent the number of sensors 26 from increasing, thereby reducing the parts count, and simplifying the configuration of the apparatus.

For the sake of simple explanation, in the specification of the present application, the configuration in which a sensor 26 (in a case of a light emitting-receiving sensor, linear light emitted from a light emitter) is arranged between the center of a tip end (an end toward the Y1 direction) of a particular pedal 20 and the center of a tip end (an end toward the Y1 direction) of a pedal 20 adjacent to the particular pedal 20 will be simply described as "a sensor is arranged between a particular pedal and a pedal adjacent thereto."

In addition, in the specification of the present application, a sensor 26 arranged near the center of a tip end (an end toward the Y1 direction) of a particular pedal 20 (or a light emitting-receiving sensor, a light emitter of which emits linear light passing the center), and a sensor arranged between the particular pedal and a pedal adjacent to the particular pedal will be referred to as "sensors arranged adjacent to a particular pedal."

Further, one of the plurality of sensors 26 which are arranged adjacent to a particular pedal 20 is arranged between the particular pedal 20 (the first pedal, i.e., the coagulation pedal 21 and the cutting pedal 22) and a pedal 20 adjacent thereto. That is, the plurality of sensors 26 arranged adjacent to the pedal 20 (the cutting pedal 22a, the coagulation pedal 21a, the cutting pedal 22b, the coagulation pedal 21b) are arranged on both sides in the width direction (the X direction) of the pedal 20 (the cutting pedal 22a, the coagulation pedal 21a, the cutting pedal 22b, the coagulation pedal 21b). The control unit 61 uses the detection information sent from the sensor 26 arranged between the particular pedal 20 and the pedal 20 adjacent to the particular pedal 20 in order to determine whether the foot operating the adjacent pedal 20 is present or not. Specifically, the control unit 61 uses the detection information sent from the sensor 26 arranged between the particular pedal 20 and the pedal 20 adjacent to the particular pedal 20 in order to determine whether the foot operating the particular pedal 20 is present or not, and whether the foot operating the pedal 20 adjacent to the particular pedal 20 is present or not.

According to the first embodiment, the foot operating at least one of the plurality of pedals 20 is detected using a plurality of sensors 26, and the foot operating at least one of the plurality of pedals 20 is detected using a single sensor 26. This configuration can prevent the number of sensors 26 from increasing, thereby reducing the parts count, and simplifying the configuration of the apparatus.

If a distance between the pedal 20 and the pedal 20 adjacent thereto is equal to or more than a predetermined distance, a single sensor 26 may be arranged near the former pedal 20. If the distance between the pedal 20 and a pedal 20 adjacent thereto is less than the predetermined distance, a plurality of sensors 26 may be arranged near the former pedal 20. That is, if the distance between the pedal 20 and the pedal 20 adjacent thereto is equal to or more than the predetermined distance, the control unit 61 determines whether the foot operating the former pedal 20 is present or not using the detection information sent from the single sensor 26. If the distance between the pedal 20 and the pedal 20 adjacent thereto is less than the predetermined distance, the control unit 61 determines whether the foot operating the former pedal 20 is present or not using the detection information sent from the plurality of sensors 26.

The predetermined distance between the pedals adjacent to each other may be set to be 30 mm or more and 40 mm or less. For example, if the distance from the pedal 20 to the pedal adjacent thereto is less than 35 mm, two sensors are arranged near the pedal 20. If the predetermined distance is equal to or more than 35 mm, only a single sensor is arranged near the pedal 20. Specifically, for example, the operation pedal unit 2 has a width of about 640 mm, in which a distance d3 is about 26 mm and a distance d4 is about 48 mm.

If the distance between the pedals 20 is small and it is difficult to distinguish the target pedal 20, use of a plurality of sensors 26 makes it possible to distinguish the target pedal 20 with accuracy. If the distance between the pedals 20 is large and it is easy to distinguish the target pedal 20, the single sensor 26 is used. This can effectively prevent the number of sensors 26 from increasing.

Specifically, a distance between the cutting pedal 22a and the coagulation pedal 21a, a distance between the coagulation pedal 21a and the cutting pedal 22b, and a distance between the cutting pedal 22b and the coagulation pedal 21b are the distance d3 which is less than the predetermined distance. That is, a distance between the first pedal (the coagulation pedal 21 or the cutting pedal 22) and the pedal 20 adjacent thereto is less than the predetermined distance. More specifically, the distance between the first pedal (the coagulation pedal 21 or the cutting pedal 22) and the pedal 20 adjacent thereto may be set to be less than 40 mm. A distance between the camera pedal 23 and the clutch pedal 24 is the distance d4 which is equal to or greater than the predetermined distance. That is, a distance between the second pedal (the camera pedal 23 or the clutch pedal 24) and the pedal 20 adjacent thereto is equal to or greater than the predetermined distance. More specifically, the distance between the second pedal (the camera pedal 23 or the clutch pedal 24) and the pedal 20 adjacent thereto may be set to be equal to or greater than 40 mm. Thus, the distance between the first pedal (the coagulation pedal 21 or the cutting pedal 22) and the pedal 20 adjacent thereto is smaller than the distance between the second pedal (the camera pedal 23 or the clutch pedal 24) and the pedal 20 adjacent thereto. In other words, the distance between the second pedal (the camera pedal 23 or the clutch pedal 24) and the pedal 20 adjacent thereto is greater than the distance between the first pedal (the coagulation pedal 21 or the cutting pedal 22) and the pedal 20 adjacent thereto. Thus, a single sensor 26 (the sensor 26f or the sensor 26g) is provided near the second pedal (the camera pedal 23 or the clutch pedal 24).

The sensors 26a and 26b detect the foot approaching the coagulation pedal 21b. Specifically, if both of the sensors 26a and 26b detect the foot, the control unit 61 determines that the target pedal 20 is the coagulation pedal 21b. The sensors 26b and 26c detect the foot approaching the cutting pedal 22b. Specifically, if both of the sensors 26b and 26c detect the foot, the control unit 61 determines that the target pedal 20 is the cutting pedal 22b.

The sensors 26c and 26d detect the foot approaching the coagulation pedal 21a. Specifically, if both of the sensors 26c and 26d detect the foot, the control unit 61 determines that the target pedal 20 is the coagulation pedal 21a. The sensors 26d and 26e detect the foot approaching the cutting pedal 22a. Specifically, if both of the sensors 26d and 26e detect the foot, the control unit 61 determines that the target pedal 20 is the cutting pedal 22a.

The sensor 26f detects the foot approaching the clutch pedal 24. Specifically, if the single sensor 26f detects the foot, the control unit 61 determines that the target pedal 20 is the clutch pedal 24 (the second pedal). The sensor 26g detects the foot approaching the camera pedal 23. Specifically, if the single sensor 26g detects the foot, the control unit 61 determines that the target pedal 20 is the camera pedal 23 (the second pedal).

The light emitter 261 includes a light emitting element such as an LED. The light emitter 261 is configured to emit visible light or invisible light such as infrared light. The light receiver 262 includes a light receiving element. The light emitter 261 and the light receiver 262 associated with each other are arranged in the Y direction when viewed in plan. Specifically, as indicated by dotted lines in FIGS. 4 to 6, the light emitter 261 emits light principally along the Y direction when viewed in plan. The light emitter 261 and the light receiver 262 associated with each other are arranged parallel to each other when viewed in plan. In this configuration, unlike a case where the associated pairs of the light emitter 261 and light receiver 262 of the sensors 26 are arranged in a fan-shaped pattern, the operation pedal unit 2 does not need to be increased in width (the dimension in the X direction) to arrange the sensors 26 at outer positions.

The light emitter 261 is configured to emit light obliquely downward.

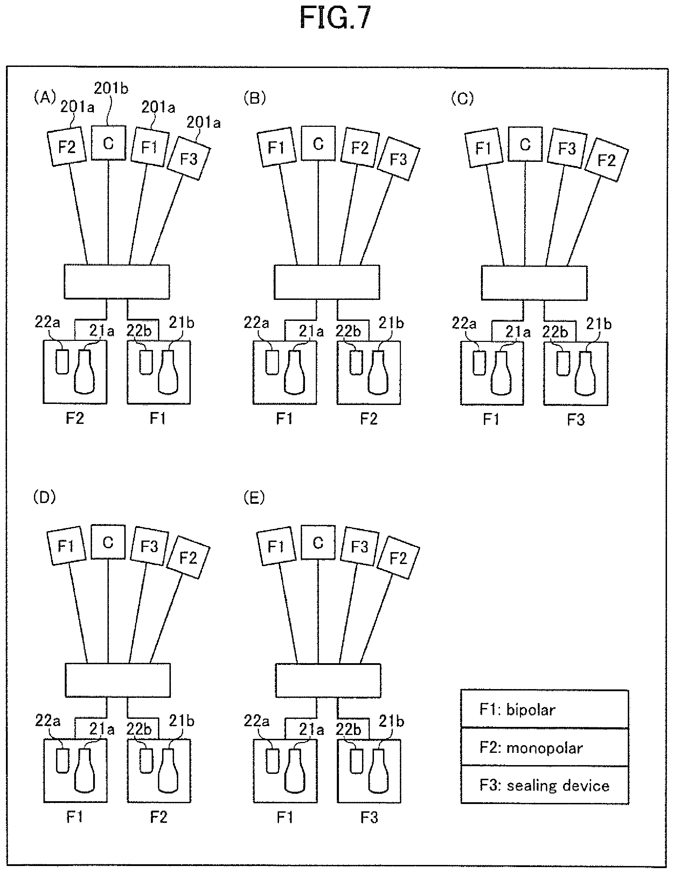

Referring to FIG. 7, an example of allocation of the coagulation pedals (21a and 21b) and the cutting pedals 22 (22a and 22b) of the operation pedal unit 2 will be described below. The coagulation pedal 21a and the cutting pedal 22a are used in a set, and the coagulation pedal 21b and the cutting pedal 22b are used in a set. Note that a surgical site can be cut or coagulated using a single forceps (e.g., a grasper). If a single forceps is used for the cutting and the coagulation, a high voltage is applied for the cutting, while a voltage lower than the voltage for the cutting is applied for the coagulation. Specifically, selectively using the coagulation pedal 21a (21b) and the cutting pedal 22a (22b) makes it possible to coagulate and cut the surgical site. If the cutting and the coagulation can be performed using a grasper or any other tool, a sealing device for the coagulation may be used exclusively or in combination. This is because the sealing device often has additional functions, e.g., of automatically stopping the power feeding when the coagulation finishes.

In the examples shown in FIG. 7, four instruments 201a, namely, a bipolar forceps F1, a monopolar forceps F2, a sealing device F3, and the endoscope 201b are attached to the four surgical manipulators 201. The positional relationship among the four surgical manipulators 201 is recognized by a position detector provided for each manipulator. The positional relationship among the manipulators in the horizontal direction is recognized with reference to the platform 203. In an example of FIG. 7(A), the monopolar forceps F2 is arranged on the left of the camera arm 201B to which the endoscope 201b is attached, and the bipolar forceps F1 and the sealing device F3 are arranged sequentially from the left on the right of the camera arm 201B. As an example of the allocation of the coagulation pedals 21 (21a and 21b) and the cutting pedals 22 (22a and 22b) will be described below. First, among the instrument arms 201A to each of which the instrument 201a is attached, the leftmost instrument arm 201A is allocated to the left foot pedals (the coagulation pedal 21a and the cutting pedal 22a), and the second leftmost instrument arm 201A is allocated to the right foot pedals (the coagulation pedal 21b and the cutting pedal 22b). That is, in the example of FIG. 7(A), the monopolar forceps F2 is allocated to the left foot pedals (the coagulation pedal 21a and the cutting pedal 22a). Likewise, the bipolar forceps F1 is allocated to the right foot pedals (the coagulation pedal 21b and the cutting pedal 22b). If there are only two instruments 201a attached, the leftmost instrument arm 201A with respect to the camera arm 201B is first allocated to the left foot pedals, and the other instrument arm 201A is allocated to the right foot pedals. If there is only a single instrument 201a attached, it is allocated to the left foot pedals.

In an example of FIG. 7(B), the bipolar forceps F1 and the monopolar forceps F2 are exchanged by an assistant (e.g., a nurse). In this case, the type of the instrument 201a is identified when the instrument 201a is attached to the instrument arm 201A. For example, the interface may store in its integrated circuit (IC) information such as a model number of the instrument. The bipolar forceps F1 is then allocated to the left foot pedals (the coagulation pedal 21a and the cutting pedal 22a). The monopolar forceps F2 is allocated to the right foot pedals (the coagulation pedal 21b and the cutting pedal 22b).

In an example of FIG. 7(C), the monopolar forceps F2 and the sealing device F3 are exchanged by an assistant (e.g., a nurse). The type of the instrument 201a is identified when the instrument 201a is attached to the instrument arm 201A. The bipolar forceps F1 is kept allocated to the left foot pedals (the coagulation pedal 21a and the cutting pedal 22a). The sealing device F3 is allocated to the right foot pedals (the coagulation pedal 21b and the cutting pedal 22b).

In an example of FIG. 7(D), the side pedal 25 is operated to switch the instrument arm 201A to be activated, between the two instrument arms 201A on the right side. Specifically, the instrument arm 201A to be operated by the operating handle 1 is changed. Thus, the instrument arm 201A to which the bipolar forceps F1 is attached is operated by the left operating handle 1, and the instrument arm 201A to which the monopolar forceps F2 is attached is operated by the right operating handle 1. The bipolar forceps F1 is kept allocated to the left foot pedals (the coagulation pedal 21a and the cutting pedal 22a). The monopolar forceps F2 is allocated to the right foot pedals (the coagulation pedal 21b and the cutting pedal 22b).

In an example of FIG. 7(E), the side pedal 25 is operated to switch the instrument arm 201A to be activated, between the two instrument arms 201A on the right side. Specifically, the instrument arm 201A to be operated by the operating handle 1 is changed. Thus, the instrument arm 201A to which the bipolar forceps F1 is attached is operated by the left operating handle 1, and the instrument arm 201A to which the sealing device F3 is attached is operated by the right operating handle 1. The bipolar forceps F1 is kept allocated to the left foot pedals (the coagulation pedal 21a and the cutting pedal 22a). The sealing device F3 is allocated to the right foot pedals (the coagulation pedal 21b and the cutting pedal 22b).

A simple grasper, which is not energized and is generally used when a great gripping force is required, may sometimes be used. Since the instrument 201a which cannot be energized is not operated by the coagulation pedals 21 (21a and 21b) and the cutting pedals 22 (22a and 22b), the coagulation pedals 21 and the cutting pedals 22 are not allocated to such an instrument. That is, the instrument arm 201A gripping the instrument 201a which cannot be energized can be ignored in setting for the allocation of the coagulation pedals 21 (21a and 21b) and the cutting pedals 22 (22a and 22b).

The left foot pedals (the coagulation pedal 21a and the cutting pedal 22a) and the right foot pedals (the coagulation pedal 21b and the cutting pedal 22b) may be allocated in accordance with other methods (rules). For example, each of the two instrument arms 201A on the left and right of the camera arm 201B may be allocated to the foot pedal. For example, the single instrument arm 201A on the left of the camera arm 201B may be allocated to the left foot pedals (the coagulation pedal 21a and the cutting pedal 22a), and the left one of the two instrument arms 201A on the right of the camera arm 201B may be allocated to the right foot pedals (the coagulation pedal 21b and the cutting pedal 22b).

If the instruments 201a and the endoscope 201b are attached to the surgical manipulators 201 such that two instrument arms 201A are present on the left of the camera arm 201B, the left one of the two instrument arms 201A on the left of the camera arm 201B may be allocated to the left foot pedals (the coagulation pedal 21a and the cutting pedal 22a), and the instrument arm 201A on the right of the camera arm 201B may be allocated to the right foot pedals (the coagulation pedal 21b and the cutting pedal 22b). In a preferred embodiment, the following rule is made in this case. If two instrument arms 201A are detected on the left of the camera arm 201B and the single instrument arm 201A is detected on the right of the camera arm 201B, the two instrument arms 201A on the left of the camera arm 201B are automatically selected as those switched by the side pedal 25.

In a preferred embodiment, if there are a plurality of instrument arms 201A on each of the right and left sides of the camera arm 201B, the instrument arms 201A on one side (e.g., the surgical manipulators 201 on the right corresponding to right handers (the number of the right handers is greater than the number of left handers)) may be set as the target of the switching by the side pedal 25. Then, the target of the switching by the side pedal 25 may be changed (between the plurality of instrument arms 201A on the right of the camera arm 201B and the plurality of instrument arms 201A on the left of the camera arm 201B) by an additional operating element (e.g., a touch panel (an operating unit 53) provided at the arm rest 5). As another example, a second side pedal (not shown) may be provided on the right of the coagulation pedal 21b such that the side pedal 25 is used for the switching among the plurality of instrument arms 201A on the left of the camera arm 201B, and the second side pedal is used for the switching among the plurality of instrument arms 201A on the right of the camera arm 201B.

In the foregoing description, it has been described as an example that the camera arm 201B is the inner one of the plurality of surgical manipulators 201. However, the camera arm 201B may be the outermost one of the plurality of surgical manipulators 201. Also in this case, according to a specific rule, for example, the leftmost instrument arm 201A may be allocated to the left foot pedals (the coagulation pedal 21a and the cutting pedal 22a), the second leftmost instrument arm 201A may be allocated to the right foot pedals (the coagulation pedal 21b and the cutting pedal 22b), and the second and third leftmost instrument arms 201A are set as the target of the switching by the side pedal 25. In a preferred embodiment, the target of the switching by the side pedal 25 may be changed by an additional operating element to, for example, the leftmost instrument arm 201A and the second leftmost instrument arm 201A, as described above.

Further, the following rule may be made in which if the camera arm 201B is detected as the inner one of the plurality of surgical manipulators 201, the instrument arm 201A adjacent to the camera arm 201B is preferentially allocated to the foot pedal. For example, in the example of FIG. 7(A), a single instrument arm 201A is present on the left of the camera arm 201B, and two instrument arms 201A are present on the right of the camera arm 201B. The single instrument arm 201A on the left of the camera arm 201B may be allocated to the left foot pedals (the coagulation pedal 21a and the cutting pedal 22a), and one of the two instrument arms 201A closer to the camera arm 201B may be allocated to the right foot pedals (the coagulation pedal 21b and the cutting pedal 22b). If there are two instrument arms 201A on the left of the camera arm 201B, one of the two instrument arms 201A closer to the camera arm 201B, i.e., the right one, may be allocated to the left foot pedals (the coagulation pedal 21a and the cutting pedal 22a), and the single instrument arm 201A on the right of the camera arm 201B may be allocated to the right foot pedals (the coagulation pedal 21b and the cutting pedal 22b).

In the foregoing description, the endoscope 201b is attached to the surgical manipulator 201 mounted on the platform 203, and the rule of the allocation is set through a recognition of the position of the camera arm 201B. However, even if the camera arm 201B is provided separately from the platform 203, the positional relationship between the camera arm 201B and the plurality of instrument arms 201A in the world coordinate system may be recognized through calibration so that the above-described rule can be used. In this case, the viewpoint from which the positional relationship among the surgical manipulators 201 in the horizontal direction is determined by the operator O or needs to be set as default.

As can be seen in the foregoing, the surgical manipulators 201 operated by the left and right operating handles 1 are allocated in accordance with the rule. This configuration can achieve simple allocation of the two sets of foot pedals to the plurality of instrument arms 201A without additionally providing a complicated detector configured, for example, to detect to which manipulator each of the left and right operating handles 1 corresponds.

In a preferred embodiment, in order that the operator O can reliably understand how the two sets of foot pedals are allocated to the left and right operating handles 1 and to the plurality of instrument arms 201A, the display unit 3 showing images from the endoscope 201b may at least show the state of the allocation of the foot pedals to the operating handles 1.

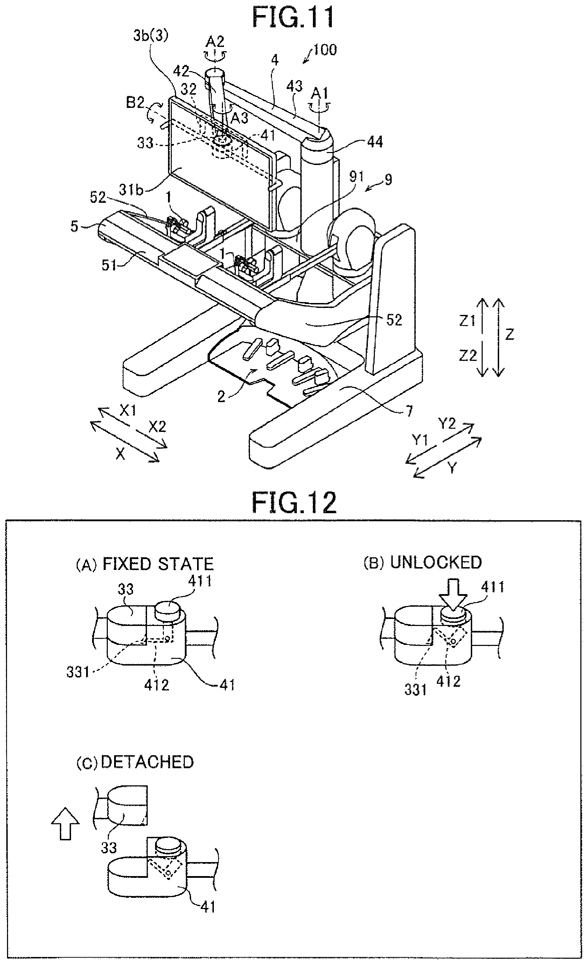

The display unit 3 can display the images taken by the endoscope 201b. The display unit 3 may include a scope type display unit 3a or a non-scope type display unit 3b. The scope type display unit 3a is, for example, a display unit which an operator looks into. The non-scope type display unit 3b is an open display unit having a flat screen which an operator does not look into, just like a display of a common personal computer. The scope type display unit 3a and the non-scope type display unit 3b are configured such that they can be selectively attached to the remote control apparatus 100. Specifically, the scope type display unit 3a includes, as shown in FIG. 2, a display 31a, a grip 32, and a mounting portion 33. The non-scope type display unit 3b includes, as shown in FIG. 11, a display 31b, a grip 32, and a mounting portion 33. The mounting portion 33 of the scope type display unit 3a or the non-scope type display unit 3b is configured such that it can be attached to a mounting counterpart 41 of the display unit supporting arm 4 of the remote control apparatus 100. Specifically, the scope type display unit 3a or the non-scope type display unit 3b attached to the remote control apparatus 100 is configured to be supported by the display unit supporting arm 4. Thus, both of an immersive remote control apparatus and an open remote control apparatus are selectable, and the remote control apparatus 100 having flexibility in terms of the display unit 3 can be provided.

Surgery generally takes several hours, and an operator may feel isolated if the operator works for a long time with an immersive remote control apparatus. In such a case, the remote control apparatus can be switched to an open type before or during the surgery so that the operator can feel that the operator is performing the surgery as one of team members.

If the remote control apparatus has flexibility and extensibility in terms of the display unit, the display unit can be repaired independently of the apparatus even when the display unit fails or is damaged. This is advantageous because the whole apparatus does not need to be replaced. As another advantage, the display unit can be upgraded every time the display unit with improved definition and quality is developed, without replacing the whole apparatus. As still another advantage, the operator O can select the display unit depending on the operator's preferred manufacturer and specification (such as size, shape, and an operation panel).

As shown in FIG. 3, the display unit 3 includes a terminal 34. Examples of the terminal 34 include a terminal capable of transmitting video, such as a serial digital interface (SDI) terminal, an analog component terminal, a High-Definition Multimedia Interface (HDMI (registered trademark)) terminal, and a universal serial bus (USB) terminal. The terminal 34 is connected to the control device 6. Specifically, when a connecting wire is connected to the terminal 34, image information is transmitted from the control device 6 to the display unit 3. When the connecting wire is disconnected from the terminal 34, the display unit 3 can be detached from the remote control apparatus 100.

In a case in which the scope type display unit 3a is attached, a 3D image taken by the endoscope 201b gripped by the camera arm 201B of the patient-side system 200 is displayed. Also in a case in which the non-scope type display unit 3b is attached, a 3D image taken by the endoscope 201b provided in the patient-side system 200 is displayed. In the case in which the non-scope type display unit 3b is attached, a 2D image taken by the endoscope 201b provided in the patient-side system 200 may be displayed.

The scope type display unit 3a is a viewer which the operator O looks into. The scope type display unit 3a displays an image for the right eye of the operator O and an image for the left eye of the operator O. The scope type display unit 3a is, for example, a stereoscope. That is, the display 31a includes a left-eye display and a right-eye display. When the operator looks into the display 31a, the left eye cannot see the right-eye display, and the right eye cannot see the left-eye display. The display 31a may be comprised of a liquid crystal display or an organic EL display. The display 31a may be a projection type display.

The non-scope type display unit 3b is an open display unit which the operator can see without looking into it. The non-scope type display unit 3b is a direct view type display unit. That is, the display 31b of the non-scope type display unit 3b has a screen with a flat or curved surface. For example, a display having a diagonal line of 10 to 90 inches may be used as the display 31b. However, taking the balance between the sufficient viewability of the surgical field and the ease of replacement of the display into consideration, a display of about 15 to 35 inches is suitable. The display 31b may be comprised of a liquid crystal display or an organic EL display. The display 31b may be a projection type display. In order that the operator O can three-dimensionally see the image taken by the endoscope 201b, any known stereoscopic system using polarizing glasses or active shutter glasses may be applied.

When the sensors 26 detect the presence of the foot of the operator O, the detection information about the presence of the foot is displayed on a foot pedal layout map shown on the display unit 3. For example, on the foot pedal layout map, the six pedals 20 operated when pressed downward are arranged in the same order as shown in FIG. 5 (in the order of the camera pedal 23, the clutch pedal 24, the cutting pedal 22a, the coagulation pedal 21a, the cutting pedal 22b, and the coagulation pedal 21b from the left in the X direction). When the presence of the foot is detected, the pedals are highlighted by changing the color, changing the depth of the color, or blinking. The display unit 3 may display (e.g., on the foot pedal layout map) the function of the target pedal 20 (e.g., coagulation, cutting, camera, and clutch). Further, the display unit 3 may display the information of the end effector operated by the corresponding pedal 20, and the information of the end effector may be highlighted when the presence of the foot is detected. The display unit 3 shows the information of the surgical manipulator 201 gripping the corresponding forceps (e.g., the number of the surgical manipulator 201). For example, if the pedal 20 corresponds to the right operating handle 1, the information of the end effector and the surgical manipulator may be shown on the right part of the display unit 3. If the pedal 20 corresponds to the left operating handle 1, the information of the end effector and the surgical manipulator may be shown on the left part of the display unit 3.

The grip 32 is gripped when the display unit 3 is attached, detached, or moved. The grip 32 can be held by one hand. For example, the grip 32 may be in the shape of a handle, a recess, or a lug. The grip 32 is provided for the side surface or back surface of the display unit 3 so as not to obstruct the operator looking at the display 31a (31b). The grip 32, which can be held by one hand, may include a plurality of grips. For example, as shown in FIG. 2, the grip 32 may be provided for each side of the display unit 3, so that the operator O sitting in front of the display unit 3 can hold the grip 32 by either of the left or right hand.

The mounting portion 33 is attached to the mounting counterpart 41 of the display unit supporting arm 4. Specifically, the scope type display unit 3a and the non-scope type display unit 3b are selectively attachable to and detachable from the mounting counterpart 41. For example, the mounting portion 33 may include an engaging portion 331, as in a first example shown in FIG. 12. The mounting counterpart 41 may include an unlock button 411 and an engaging portion 412. In a fixed state shown in FIG. 12(A), the engaging portion 331 of the mounting portion 33 and the engaging portion 412 of the mounting counterpart 41 engage with each other, thereby fixing the mounting portion 33 to the mounting counterpart 41 of the display unit supporting arm 4. The display unit 3 is fixed and supported onto the display unit supporting arm 4 in this manner. Specifically, the engaging portions 331 and 412 constitute a lock mechanism for fixing the display unit 3 (the scope type display unit 3a or the non-scope type display unit 3b).

As shown in FIG. 12(B), when the unlock button 411 is pressed downward, the engaging portion 412 moves to release the engagement between the engaging portions 331 and 412. The fixed state (locked state) of the mounting portion 33 and the mounting counterpart 41 is released in this manner. Specifically, the unlock button 411 functions as an unlock mechanism which releases the fixed state achieved by the lock mechanism constituted of the engaging portions 331 and 412. The unlock mechanism is configured to release the fixed state achieved by the lock mechanism by the action of a vertically downward force. This allows the unlock mechanism to easily release the fixed state achieved by the lock mechanism.

As shown in FIG. 12(C), the display unit 3 is detached from the remote control apparatus 100 when an upward force in the vertical direction is acted on the grip 32 of the display unit 3 while the downward force in the vertical direction is being acted on the unlock mechanism. Thus, the display unit 3 can be detached stably and safely because the display unit 3 is detached by utilizing the opposing forces, i.e., the vertically downward force for releasing the fixed state and the vertically upward force for lifting the operating unit. The display unit 3 can be separated in the vertical direction away from the display unit supporting arm 4, which makes it possible to detach the display unit 3 without interference with the operating handle 1 located below the display unit 3.

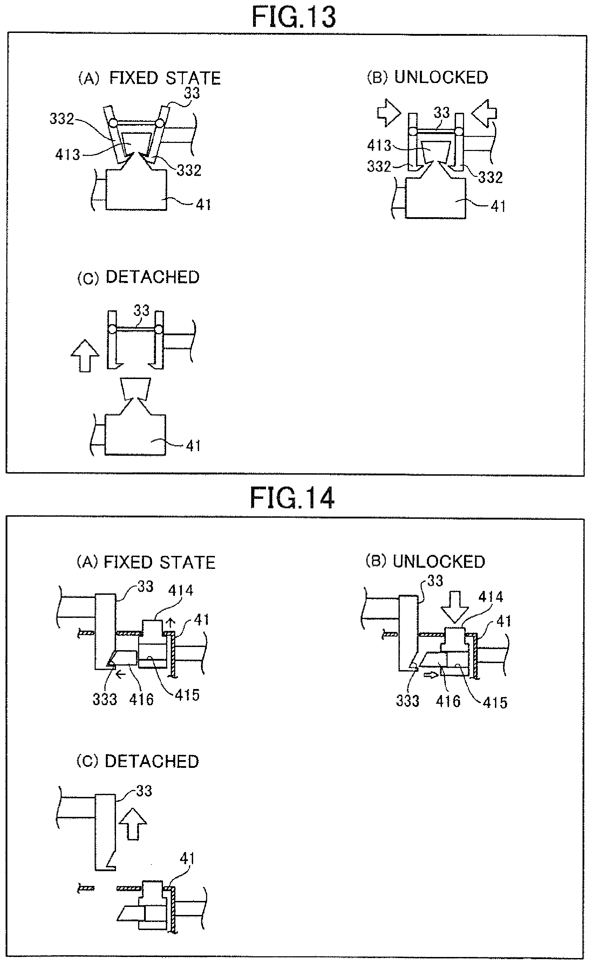

The lock mechanism and the unlock mechanism may be configured in a different manner. For example, these mechanisms may be configured as in a second example shown in FIG. 13. The mounting portion 33 includes an engaging portion 332, as in the second example shown in FIG. 13. The mounting counterpart 41 includes an engaging portion 413. In a fixed state shown in FIG. 13(A), the engaging portion 332 of the mounting portion 33 and the engaging portion 413 of the mounting counterpart 41 engage with each other, thereby fixing the mounting portion 33 to the mounting counterpart 41 of the display unit supporting arm 4. Specifically, the engaging portion 332 sandwiches and grasps the engaging portion 413 for engagement. The display unit 3 is fixed and supported onto the display unit supporting arm 4 in this manner. Specifically, the engaging portions 332 and 413 constitute a lock mechanism for fixing the display unit 3 (the scope type display unit 3a or the non-scope type display unit 3b).

As shown in FIG. 13(B), when the engaging portion 332 is pressed from both sides, the engaging portion 332 no longer sandwiches the engaging portion 413, thereby releasing the engagement between the engaging portions 332 and 413. Thus, the fixed state (locked state) of the mounting portion 33 and the mounting counterpart 41 is released. As shown in FIG. 13(C), the display unit 3 is detached from the remote control apparatus 100 when a vertically upward force is acted on the grip 32 of the display unit 3 while the fixed state is released.

The lock mechanism and the unlock mechanism may be configured in a different manner. For example, these mechanisms may be configured as in a third example shown in FIG. 14. The mounting portion 33 may include a notch 333 as in the third example shown in FIG. 14. The mounting counterpart 41 may include an unlock button 414, a fitting portion 415, and an engaging portion 416. As shown in FIG. 14(A), the unlock button 414 is biased upward in the vertical direction by a spring or any other means. The engaging portion 416 is biased in a direction away from the fitting portion 415 in the horizontal direction. A gear or any other means interlocks the vertical movement of the unlock button 414 and the horizontal movement of the engaging portion 416.

In a fixed state, the notch 333 of the mounting portion 33 and the engaging portion 416 of the mounting counterpart 41 engage with each other, thereby fixing the mounting portion 33 to the mounting counterpart 41 of the display unit supporting arm 4. The display unit 3 is fixed and supported onto the display unit supporting arm 4 in this manner. Specifically, the notch 333 and the engaging portion 416 constitute a lock mechanism for fixing the display unit 3 (the scope type display unit 3a or the non-scope type display unit 3b).

As shown in FIG. 14(B), when the unlock button 414 is pressed downward, the fitting portion 415 moves downward. Along with this movement, the engaging portion 416 moves toward the fitting portion 415, and fits into the fitting portion 415. This releases the engagement between the notch 333 and the engaging portion 416. As a result, the fixed state (locked state) of the mounting portion 33 and the mounting counterpart 41 is released. Specifically, the unlock button 414 functions as an unlock mechanism which releases the fixed state achieved by the lock mechanism constituted of the notch 333 and the engaging portion 416. The unlock mechanism is configured to release the fixed state achieved by the lock mechanism by the action of a vertically downward force.

As shown in FIG. 14(C), the display unit 3 is detached from the remote control apparatus 100 when an upward force in the vertical direction is acted on the grip 32 of the display unit 3 while the fixed state is released.

The engaging portion 416 has an inclined surface so that the lower dimension is greater than the upper dimension. Thus, when the mounting portion 33 is pushed downward in the vertical direction toward the mounting counterpart 41, the mounting portion 33 abuts the inclined surface of the engaging portion 416 and pushes the engaging portion 416 in the horizontal direction toward the fitting portion 415. When the mounting portion 33 moves to a predetermined position, the engaging portion 416 fits into the notch 333 to bring the mounting portion 33 and the mounting counterpart 41 in the fixed state.

The display unit supporting arm 4 is configured to support the display unit 3 as shown in FIG. 2. The display unit supporting arm 4 includes the mounting counterpart 41 and arms 42 and 43. One end of the display unit supporting arm 4 is provided with the mounting counterpart 41, and the other end thereof is supported by a column 44. The column 44 is fixed to the support 91 of the support mechanism 9. That is, the display unit 3 is supported by the support 91. The display unit supporting arm 4 supports the mounting counterpart 41 such that the mounting counterpart 41 is rotatable about rotational axes A1, A2, and A3 extending in the vertical direction. That is, the mounting counterpart 41 is supported by support members each having a vertical rotational axis, which allows an angular adjustment with three degrees of freedom. Specifically, the arm 43 is supported to be rotatable about the rotational axis A1 in the horizontal direction with respect to the column 44. The arm 42 is supported to be rotatable about the rotational axis A2 in the horizontal direction with respect to the arm 43. The mounting counterpart 41 is supported to be rotatable about the rotational axis A3 in the horizontal direction with respect to the arm 42. Thus, the display unit 3 attached to the mounting counterpart 41 can move in the horizontal direction. This allows the operator O to arrange the display unit 3 at a desired position.

In a case in which the scope type display unit 3a is attached to the remote control apparatus 100, the scope type display unit 3a is configured to tilt and rotate about a rotational axis B1, extending in the horizontal direction and substantially orthogonal to the rotational axis A3, as shown in FIG. 2. In a case in which the non-scope type display unit 3b is attached to the remote control apparatus 100, the non-scope type display unit 3b is configured to tilt and rotate about a rotational axis B2, extending in the horizontal direction and substantially orthogonal to the rotational axis A3, as shown in FIG. 11. This configuration can regulate an angle of elevation and an angle of depression of the display unit 3 attached to the mounting counterpart 41. The orientation of the display unit supporting arm 4 may be changed manually by the operator O or any other person, or by control of a driving unit including a motor, an encoder, and a brake.

The arm rest 5 is configured to support the arms of the operator O. The arm rest 5 includes an arm supporting portion 51, a pair of connectors 52, and an operating unit 53. The arm supporting portion 51 is arranged forward (toward the Y1 direction) of the operating handle 1 and supports the arms of the operator O. This configuration can stabilize the arms of the operator O, and therefore allows the operator O to stably control the operating handle 1. Specifically, even in the case where a fine movement of the end effector is required, the operator O can stably operate the end effector with the elbows resting on the arm rest 5. This can reduce the burden of the operator O even if the surgery takes long time. The arm supporting portion 51 is formed to extend in the X direction. A pair of connectors 52 is provided. The pair of connectors 52 is respectively arranged on the sides of the arm supporting portion 51 to sandwich the arm supporting portion 51 in the X direction. The connectors 52 are configured to support the arm supporting portion 51. The connectors 52 are formed to extend in the Y direction. That is, each of the connectors 52 has an end toward the Y1 direction connected to the arm supporting portion 51. Further, each of the connectors 52 has an end toward the Y2 direction connected to the support 91 of the support mechanism 9. Thus, the arm rest 5 is supported by the support mechanism 9. The connectors 52 are formed to extend upward in a direction from the depth (the Y2 direction) to the front (the Y1 direction). Thus, portions of the connectors 52 connected to the base 7 can be positioned at a lower level in the vertical direction, which can stabilize the arm rest 5. The operating unit 53 can change the setting of the remote control apparatus 100. For example, the operating unit 53 can change the posture of the remote control apparatus 100. In this case, the operating unit 53 functions as a posture changer 8.

As shown in FIG. 3, the control device 6 includes, for example, the control unit 61 including an arithmetic unit such as a CPU, a storage unit 62 including a memory such as a ROM and a RAM, and an image control unit 63. The control device 6 may be comprised of a single controller which provides centralized control, or may be configured as a plurality of controllers which work in cooperation with each other and provide distributed control. The control unit 61 determines whether the movement type instruction input through the operating handle 1 is a movement type instruction to be executed by the instrument arm 201A, or a movement type instruction to be executed by the endoscope 201b, in accordance with the state of the operation pedal unit 2. If the control unit 61 determines that the movement type instruction input through the operating handle 1 is a movement type instruction to be executed by the instrument 201a, the control unit 61 transmits this movement type instruction to the instrument arm 201A. In response, the instrument arm 201A is driven, as a result of which the operation of the instrument 201a attached to the instrument arm 201A is controlled.

Alternatively, if the control unit 61 determines that the movement type instruction input through the operating handle 1 is a movement type instruction to be executed by the endoscope 201b, the control unit 61 transmits the movement type instruction to the camera arm 201B. In response, the camera arm 201B is driven, as a result of which the operation of the endoscope 201b attached to the camera arm 201B is controlled.

Further, the control unit 61 receives the detection information about the presence of the foot of the operator O from the sensors 26, and determines whether the foot of the operator O is present or not. When the control unit 61 determines that the foot of the operator O is present, the control unit 61 controls the display unit 3 so that corresponding information is shown.

The storage unit 62 stores, for example, control programs corresponding to the types of the instruments 201a. The control unit 61 reads the control program in accordance with the type of the instrument 201a attached, thereby allowing the movement instruction entered through the operating handle 1 and/or operation pedal unit 2 of the remote control apparatus 100 to instruct each instrument 201a to perform a suitable operation.

The image control unit 63 transmits images taken by the endoscope 201b to the terminal 34 of the display unit 3. The image control unit 63 processes or corrects the image as needed.

The remote control apparatus 100 is configured such that the operating handle 1 is movable in the vertical direction as shown in FIGS. 8 and 9. Specifically, the posture changer 8 receives an instruction for a vertical movement of the operating handle 1. In accordance with the instruction received by the posture changer 8, the support mechanism 9 moves the operating handle 1 in the vertical direction.

The support mechanism 9 includes the support 91 and a driving unit 92. The support 91 supports the operating handle 1 and the arm rest 5. Further, the support 91 supports the display unit 3 via the display unit supporting arm 4. The driving unit 92 is configured to move the support 91 in the vertical direction. Specifically, the driving unit 92 includes, for example, a motor and an encoder, and moves the support 91 in the vertical direction under the control of the control unit 61. The posture of the support mechanism 9 may be manually changed by the operator O or any other person. The driving unit 92 of the support mechanism 9 may be pneumatically or hydraulically actuated. The arm rest 5 may rotate with respect to the support mechanism 9 for adjustment of the position. For example, the arm rest 5 may rotate about a rotational axis extending along the X direction.

For example, the support mechanism 9 is configured to be capable of transforming between a first posture (see FIG. 8) in which the operating handle 1 at a center position A0 of the operation area A is positioned and held at a vertical position H1 located at a height of 85 cm or more from a floor surface where the remote control apparatus 100 is placed, and a second posture (FIG. 9) in which the operating handle 1 is shifted down by 48 cm or more from the vertical position H1 to a vertical position H2 at which the operating handle 1 at the center position A0 of the operation area A is positioned and held. This configuration, in which the operating handle 1 at the center position A0 of the operation area A is positioned at the vertical position HI, i.e., 85 cm or more from the floor surface, allows the operator O to operate the operating handle 1 in a standing position. Further, the configuration in which the operating handle 1 at the center position A0 of the operation area A is shifted down by 48 cm or more from the vertical position H1 to the vertical position H2 allows the operator O to operate the operating handle 1 in a seated position. The operator O can operate the remote control apparatus 100 in a desired position. Since the support mechanism 9 supports the operating handle 1, the operator O does not need to support the operating handle 1, which may reduce an increase of a burden on the operator O. Further, the arm rest 5 supporting the arms of the operator O can further reduce the burden on the operator O, and can stabilize the arms of the operator O, allowing the operator O to stably operate the operating handle 1.

The support mechanism 9 is configured to be capable of transforming between a first posture (see FIG. 8) in which the operating handle 1 is held such that the operation area A of the operating handle 1 is included in a clean zone that is set at and above a predetermined height from a floor surface where the remote control apparatus 100 is placed, and a second posture (FIG. 9) in which the operating handle 1 is held such that at least part of the operation area A of the operating handle 1 is located in a zone below the clean zone.

In an operating room, a cleaning procedure is carried out to prevent surgery incisions and medical equipment from being infected and contaminated with pathogenic bacteria or foreign substances. A clean zone and a contaminated zone, which is a zone other than the clean zone, are defined in this cleaning procedure. A zone covering a range with a certain height H from the floor surface where foreign substances, e.g., dust or dirt, are highly likely to be present is generally treated as the contaminated zone, and excluded from the clean zone. For example, the contaminated zone covers a range up to a height of about 70 cm from the floor surface. In other words, a zone at or above the height of about 70 cm or more from the floor surface where the remote control apparatus 100 is placed is defined as the clean zone, for example. Surgery team members, including the operator O, take good care so that only a disinfected object is placed in the clean zone during the surgery, and sterilize the object placed in the contaminated zone when it needs to be moved to the clean zone. Similarly, if the surgery team members, including the operator O, have put their hands in the contaminated zone, they sterilize their hands before they directly touch an object placed in the clean zone. The operating handle 1 is not treated as a clean object. Even if the operating handle 1 is in the clean zone, the operator O never makes access to the patient P while operating the operating handle 1 unless the operating handle 1 is sterilized or draped.

Thus, positioning the operating handle 1 such that the operation area A of the operating handle 1 is included in the clean zone that is set at and above a predetermined height from the floor surface allows the operator O to operate the operating handle 1 without moving hands out of the clean zone. Such positioning of the operating handle 1 makes it possible to keep the hands of the operator O clean if, for example, the operating handle 1 is kept clean. Further, since the operating handle 1 is held such that at least part of the operation area A of the operating handle 1 is located in a zone below the clean zone, the operator O can operate the operating handle 1 at a low position, i.e., in a seated position. The operator O can operate the remote control apparatus 100 in a desired position. Since the support mechanism 9 supports the operating handle 1, the operator O does not need to support the operating handle 1, which may reduce an increase of a burden on the operator O.

The support mechanism 9 is configured to be capable of transforming between a first posture (see FIG. 8) in which the operating handle 1 is held at a position suitable for the operator O to operate the operating handle 1 in a standing position, and a second posture (see FIG. 9) in which the operating handle 1 is held at a position suitable for the operator O to operate the operating handle 1 in a seated position. Transforming the remote control apparatus 100 to the first posture allows the operator O to operate the operating handle 1 in a standing position. Transforming the remote control apparatus 100 to the second posture allows the operator O to operate the operating handle 1 in a seated position. The operator O can operate the remote control apparatus 100 in a desired position. Since the support mechanism 9 supports the operating handle 1, the operator O does not need to support the operating handle 1, which may reduce an increase of a burden on the operator O.

The support mechanism 9 is configured to be able to move both of the operating handle 1 and the arm rest 5 in the vertical direction between the first and second postures. Specifically, the support mechanism 9 is configured to move both of the operating handle 1 and the arm rest 5 together in the vertical direction between the first and second postures. This can reduce the parts count, and hence can simplify the configuration of the apparatus and avoid an increase in size of the apparatus, as compared with the case where members for respectively moving the operating handle 1 and the arm rest 5 up and down are provided. The support mechanism 9 is also configured to be able to move the display unit 3 supported by the display unit supporting arm 4 in the vertical direction between the first and second postures. That is, the support mechanism 9 is able to move the operating handle 1, the arm rest 5, and the display unit 3 together in the vertical direction between the first and second postures.

In other words, the support mechanism 9 supports the display unit 3, which displays images taken by the endoscope 201b, such that a relative position of the display unit 3 with respect to the operating handle 1 is changeable in each of the first and second postures. Specifically, the display unit supporting arm 4 supported by the support mechanism 9 changes the position of the display unit 3 with respect to the operating handle 1. This means that the position of the display unit 3 is changeable with respect to the operating handle 1 according to the physique and posture of the operator O, which can enhance the flexibility in terms of the display unit 3.

The posture changer 8 is configured to receive an instruction to move the operating handle 1, the display unit 3 supported by the display unit supporting arm 4, and the arm rest 5, in the vertical direction. The posture changer 8 is also configured to receive an instruction to move the operation pedal unit 2 in the forward and backward directions (the Y directions). Specifically, the posture changer 8 is configured to receive an instruction to transform the remote control apparatus 100 between the first and second postures.

That is, the posture changer 8 is an operating unit configured to receive a posture change instruction to change the posture of the remote control apparatus 100 between a standing position posture (a first posture) and a seated position posture (a second posture). The posture changer 8 includes a plurality of operation buttons.

The support mechanism 9 is configured to move the operating handle 1, the display unit 3 supported by the display unit supporting arm 4, and the arm rest 5, in the vertical direction. The driving unit 92 of the support mechanism 9 includes, for example, a motor and an encoder, and drives the support mechanism in accordance with an instruction from the posture changer 8. The driving unit 92 is supported by the base 7. Further, the driving unit 92 is arranged near the end of the base 7 toward the Y2 direction in the forward and backward directions (the Y directions), and substantially at the center of the base 7 in the left and right directions (the X directions). The support mechanism 9 may be configured to move the operating handle 1, the display unit 3 supported by the display unit supporting arm 4, and the arm rest 5 independently from one another in the vertical direction.

In a preferred embodiment, the support mechanism 9 in the first posture may hold the operating handle 1 so that the operating handle 1 at the center position A0 of the operation area A can be positioned at the first vertical position H1 located at a height of 99 cm or more from the floor surface where the remote control apparatus 100 is placed. Further, in a preferred embodiment, the support mechanism 9 in the second posture may shift the operating handle 1 down by 50 cm or more from the first vertical position H1 to the second vertical position H2 at which the operating handle 1 at the center position A0 of the operation area A is positioned and held.

While the postures are changed between the first and second postures, the operation of the patient-side system 200 using the operating handle 1 is invalidated. Specifically, while the postures are changed between the first and second postures, the operation of the operating handle 1 is invalidated, or the transmission of the movement type instruction is invalidated. That is, during the shift between the first and second postures, the control unit 61 does not transmit the movement type instruction to the patient-side system 200 even if the instruction is transmitted from the operating handle 1. This configuration contributes to preventing the patient-side system 200 from being operated by an unintentional operation of the operating handle 1 during the transformation between the first and second postures.

When the remote control apparatus 100 is in the standing position posture (the first posture) as shown in FIG. 8, the operating handle 1 is located at a vertical position suitable for the operator O in the standing position to grip the operating handle 1 at the center position A0 with the arms of the operator O bent at approximately right angles. In addition, the display unit 3 is located at a vertical position suitable for the operator O in the standing position to view the image on the display unit 3. For example, when the scope type display unit 3a is attached, the scope type display unit 3a is located at the vertical position of the eyes of the operator O.

In an operating room, where a zone covering a range with a height H of up to 70 cm from the floor surface is specified as the contaminated zone, the remote control apparatus 100 may be configured based on an ergonomics human model such that the entire operation area A of the operating handle 1 in the standing position posture (the first posture) is included in a clean zone, which may be located at and above a height of 70 cm or more from the floor surface.

When the remote control apparatus 100 is in the standing position posture (the first posture), the operation pedal unit 2 is moved to a position P1 on the front side (toward the Y1 direction) of the remote control apparatus 100. Specifically, the operation pedal unit 2 moves to a position suitable for the operator O who is touching the operating handle 1 by hand in the standing position to reach the operation pedal unit 2 by foot.

When the remote control apparatus 100 is in the seated position posture (the second posture) as shown in FIG. 9, the operating handle 1 is located at a vertical position suitable for the operator O sitting on a chair to grip the operating handle 1 at the center position A0 with the arms of the operator O bent at approximately right angles. In addition, the display unit 3 is located at a vertical position suitable for the operator O sitting on a chair to view the image on the display unit 3. For example, when the scope type display unit 3a is attached, the scope type display unit 3a is located at the vertical position of the eyes of the operator O. In the case of a long surgery, performing a surgery in a seated position will reduce the accumulation of fatigue of the operator O who performs the surgery.

In an operating room, where a zone covering a range with a height H of up to 70 cm from the floor surface is specified as the contaminated zone, the remote control apparatus 100 may be configured based on an ergonomics human model such that at least part of the operation area A of the operating handle 1 is included in the contaminated zone in the seated position posture (the second posture).

When the remote control apparatus 100 is in the seated position posture (the second posture), the operation pedal unit 2 is moved to a position P2 on the back side (toward the Y2 direction) of the remote control apparatus 100. Specifically, the operation pedal unit 2 moves to a position suitable for the operator O who is touching the operating handle 1 by hand in the seated position to reach the operation pedal unit 2 by foot. For example, the operation pedal unit 2 is configured to be movable by 300 mm or more in the forward and backward directions (the Y directions). In a preferred embodiment, the operation pedal unit 2 is configured to be movable by 350 mm or more in the forward and backward directions (the Y directions). This configuration allows the operation pedal unit 2 to be easily moved to a position suitable for each of the first posture and the second posture.

Measurement data described in "1988 ANTHROPOMETRIC SURVEY OF U.S. ARMY PERSONNEL: METHODS AND SUMMARY STATISTICS (1988)" was used for determining dimensions of the remote control apparatus 100.

Japanese Industrial Standards (JIS) may be used as a reference for determining dimensions of the remote control apparatus 100. For example, "JIS Z8503-4: 2006 (ISO 11064-4: 2004) Ergonomic design of control center - Part 4: Layout and dimensions of workstations" specifies using the 5th percentile and the 95th percentile of human models.

The operation area A is defined as extending upward and downward, 15 cm each, from the center position A0. In other words, the dimension of the operation area A in the height direction is defined to be 30 cm. The dimension of the operation area A in the height direction is defined based on the height dimension of the movement area of a surgical instrument which is defined to maintain satisfactory manipulation of the surgical instrument during a laparoscopic surgery, as well as based on the movement scale factor of the operating handle 1. The defined movement area for the surgical instrument has a height dimension of 30 cm, and the movement scale factor of the operating handle 1 is 1/2. Accordingly, the height dimension of the operation area A derived from the height dimension of the movement area of the surgical instrument and the movement scale factor of the operating handle 1 is 30 cm.

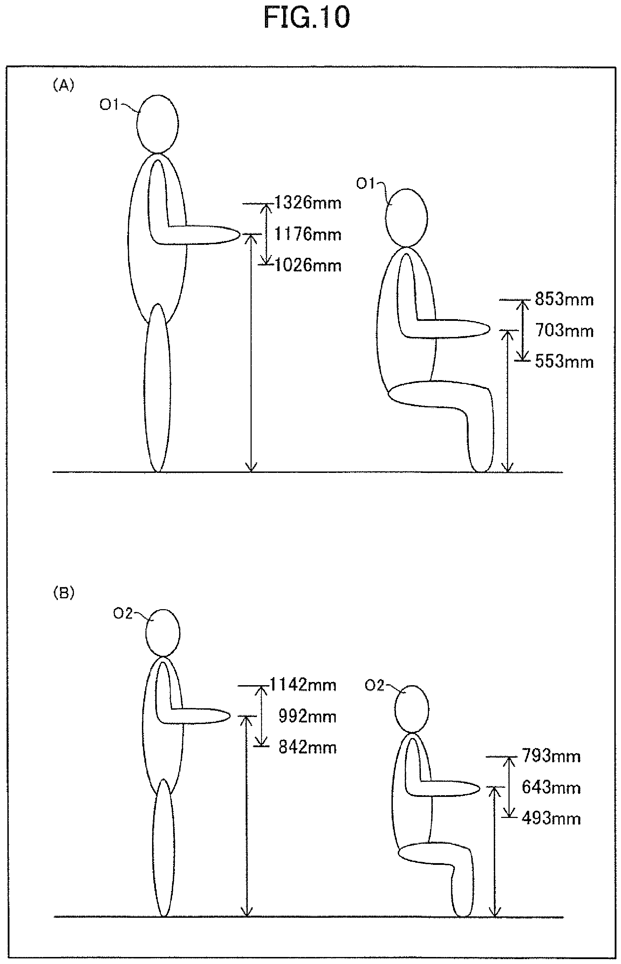

FIG. 10(A) illustrates a model operator O, specifically a large model operator O1. FIG. 10(B) illustrates another model operator O, specifically a small model operator O2.

Body size data of German men were used as the body size data of the large model operator O1 shown in FIG. 10(A). From a group of 100 randomly selected German male models, the fifth largest man model was selected as the model operator O1. In a condition in which the model operator O1 holds the operating handle 1 at the center position A0 of the operation area A with his arms bent at right angles while in the stand-up position (i.e., the standing position), the vertical position of the operating handle 1 is about 1176 mm, and the lower and upper limits of the vertical position of the operation area A are about 1026 mm and about 1326 mm, respectively. On the other hand, in a condition in which the model operator O1 holds the operating handle 1 at the center position A0 of the operation area A with his arms bent at right angles while sitting on a chair, the vertical position of the operating handle 1 is about 703 mm, and the lower and upper limits of the vertical position of the operation area A are about 553 mm and about 853 mm, respectively.

Turning to FIG. 10(B), body size data of Japanese women were used as the body size data of the small model operator O2. From a group of 100 randomly selected Japanese female models, the fifth smallest female model was selected as the model operator O2. In a condition in which the model operator O2 holds the operating handle 1 at the center position A0 of the operation area A with her arms bent at right angles while in the stand-up position, the vertical position of the operating handle 1 is about 992 mm, and the lower and upper limits of the vertical position of the operation area A are about 842 mm and about 1142 mm, respectively. On the other hand, in a condition in which the model operator O2 holds the operating handle 1 at the center position A0 of the operation area A with her arms bent at right angles while sitting on a chair, the vertical position of the operating handle 1 is about 643 mm, and the lower and upper limits of the vertical position of the operation area A are about 493 mm and about 793 mm, respectively.

Based on these data, the vertical positions of the operating handle 1 at which a plurality of operators O in different body sizes can smoothly take the standing position or the seated position are as follows. First, in a preferred embodiment, the vertical position of the operating handle 1 at the center position A0 of the operation area A in the standing position posture (the first posture) is set to be about 99 cm or more to correspond to the small model operator O2 in the standing position. This configuration allows almost all operators O in the standing position to use the operating handle 1 comfortably. In this case, in which the operating handle 1 is configured to be able to move downward by 15 cm from the center position A0, the lower limit of the vertical position of the operation area A of the operating handle 1 in the standing position posture is 84 cm or more, as described above.

In a preferred embodiment, the vertical position of the operating handle 1 at the center position A0 of the operation area A in the standing position posture (the first posture) is set to be about 85 cm or more from the floor surface. In the above-disclosed configuration, in which the operating handle 1 is configured to be able to move downward by 15 cm from the center position A0, the lower limit of the vertical position of the operation area A of the operating handle 1 in the standing position posture is 70 cm or more, meaning that the operation area A of the operating handle 1 is included in the clean zone. The lower limit of the vertical position of the operation area A which corresponds to the small model operator O2 in the standing position is about 84 cm, as mentioned above. Hence, much more operators O in different body sizes can operate the operating handle 1 comfortably in the standing position by setting the lower limit of the vertical position of the operation area A to be 70 cm above the floor surface.

Next, in a preferred embodiment, the vertical position of the operating handle 1 at the center position A0 of the operation area A in the seated position posture (the second posture) is set to be about 64 cm or more to correspond to the small model operator O2 in the seated position. Such a configuration allows almost all operators O in the seated position to use the operating handle 1 comfortably.

Next, in a preferred embodiment, the displacement (i.e., an adjustment range) of the vertical position of the operating handle 1 when the remote control apparatus 100 transforms between the standing position posture and the seated position posture is equal to or more than about 35 cm, which is the difference between about 99 cm that is the vertical position of the operating handle 1 at the center position A0 corresponding to the small model operator O2 in the standing position, and about 64 cm that is the vertical position of the operating handle 1 at the center position A0 corresponding to the small model operator O2 in the seated position.

Further, in a preferred embodiment, the displacement of the vertical position of the operating handle 1 when the remote control apparatus 100 transforms between the standing position posture and the seated position posture is equal to or more than about 48 cm, which is the difference between about 118 cm that is the vertical position of the operating handle 1 at the center position A0 corresponding to the large model operator O1 in the standing position (in other words, the highest position of the operating handle 1 at the center position A0 in the standing position posture when the operating handle 1 is used by this large model operator O1) and about 70 cm that is the vertical position of the operating handle 1 at the center position A0 corresponding to the large model operator O1 in the seated position.

As can be seen from the above described examples, the adjustment range of the vertical position of the operating handle 1 when the remote control apparatus 100 transforms between the standing position posture and the seated position posture is larger than the adjustment range that is desirably ensured so that the remote control apparatus 100 in the standing position posture can be adjusted to the body size of the operator O. For example, the adjustment range may be larger than about 19 cm representing the difference between the vertical position of the operating handle 1 at the center position A0 corresponding to the large model operator O1 and the vertical position of the operating handle 1 at the center position A0 corresponding to the small model operator O2. The adjustment range of the vertical position of the operating handle 1 when the remote control apparatus 100 transforms between the standing position posture and the seated position posture may also be larger than the adjustment range that is desirably ensured so that the remote control apparatus 100 in the seated position posture can be adjusted to the body size of the operator O. For example, the adjustment range may be larger than about 6 cm representing the difference between the vertical position of the operating handle 1 at the center position A0 corresponding to the large model operator O1 and the vertical position of the operating handle 1 at the center position A0 corresponding to the small model operator O2.

The above-described adjustment range may be further increased in a condition in which the position of the operating handle 1 is set to be higher than about 118 cm representing the vertical position of the operating handle 1 at the center position A0 corresponding to the large model operator O1 in the standing position. In a preferred embodiment, the adjustment range may be equal to or more than 50 cm from the vertical position of the operating handle 1 in the standing position posture. Further, in a preferred embodiment, the displacement of the vertical position of the operating handle 1 when the remote control apparatus 100 transforms between the standing position posture and the seated position posture may be equal to or more than about 54 cm, which is the difference between about 118 cm that is the vertical position of the operating handle 1 at the center position A0 corresponding to the large model operator O1 in the standing position and about 64 cm, which is the vertical position of the operating handle 1 at the center position A0 corresponding to the small model operator O2 in the seated position. In one or more embodiments as disclosed herein above, the operation area A may be defined as having a vertical width of 30 cm. However, the dimensions of the operation area A may be changed to have a vertical width of, e.g., 20 cm, 25 cm, or 35 cm, with the size of the operating handle 1, for example, taken into consideration.

### (Target Pedal Identification Process)

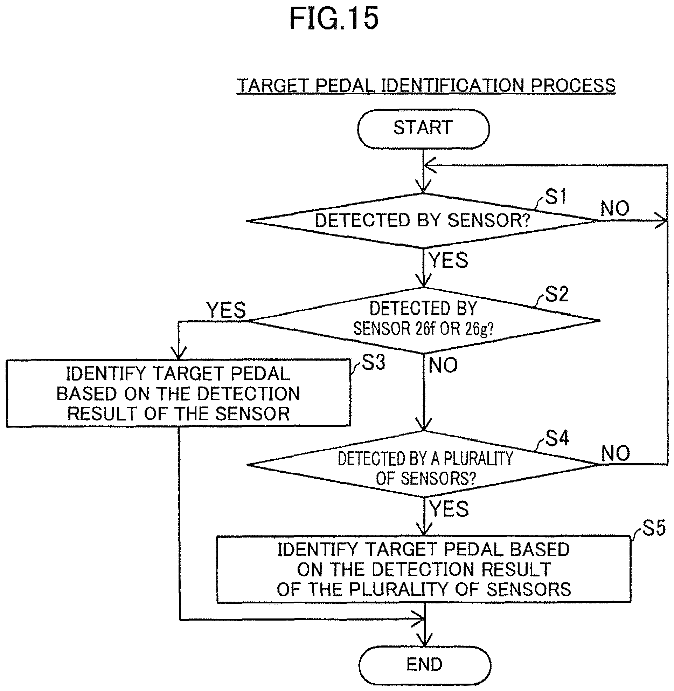

Referring to FIG. 15, a target pedal identification process performed by the control unit 61 will be described below.

In Step S1 shown in FIG. 15, the control unit 61 determines whether the sensors 26 have detected the foot of the operator O or not. If the foot of the operator O has been detected, the process proceeds to Step S2. If the foot of the operator O is not detected, the determination process in Step S1 is repeated. In Step S2, the control unit 61 determines whether the sensor 26f or the sensor 26g has detected the foot of the operator O or not. If the sensor 26 which has detected the foot is the sensor 26f or 26g, the process proceeds to Step S3. If the sensor 26 which has detected the foot is not the sensors 26f and 26g, the process proceeds to Step S4.

In Step S3, the control unit 61 identifies the target pedal 20 to be operated (the camera pedal 23 or the clutch pedal 24) based on the result of the detection of the sensor 26 (the sensor 26f or 26g). Specifically, if the sensor 26f has detected the foot, the control unit 61 identifies the clutch pedal 24 as the target pedal 20. If the sensor 26g has detected the foot, the control unit 61 identifies the camera pedal 23 as the target pedal 20.

In Step S4, the control unit 61 determines whether the plurality of sensors 26 have detected the foot of the operator O or not. If the plurality of sensors 26 have detected the foot of the operator O, the process proceeds to Step S5. If one of the sensors 26 has detected the foot of the operator O, the process returns to Step S1.

In Step S5, the control unit 61 identifies the target pedal 20 to be operated (the cutting pedal 22a, the coagulation pedal 21a, the cutting pedal 22b, or the coagulation pedal 21b) based on the result of the detection by the plurality of sensors 26. Specifically, if the plurality of sensors 26, namely, the sensors 26a and 26b, has detected the foot, the control unit 61 identifies the coagulation pedal 21b as the target pedal 20. If the plurality of sensors 26, namely, the sensors 26b and 26c, has detected the foot, the control unit 61 identifies the cutting pedal 22b as the target pedal 20. If the plurality of sensors 26, namely, the sensors 26c and 26d, has detected the foot, the control unit 61 identifies the coagulation pedal 21a as the target pedal 20. If the plurality of sensors 26, namely, the sensors 26d and 26e, has detected the foot, the control unit 61 identifies the cutting pedal 22a as the target pedal 20.

### [Second Embodiment]

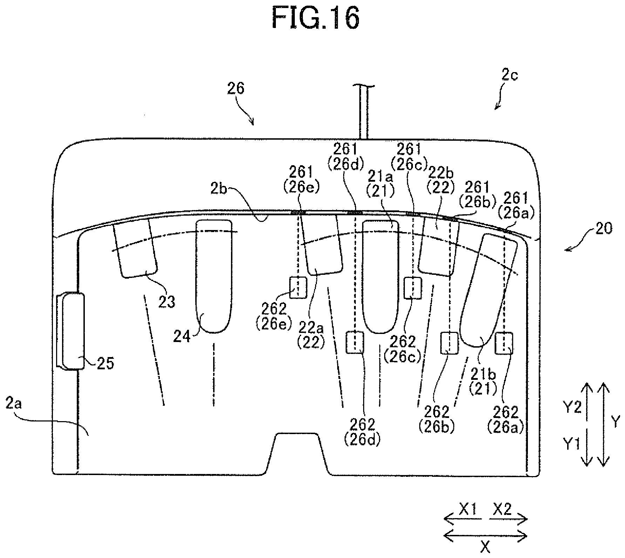

Next, referring to FIG. 16, a second embodiment of the present invention will be described below. Unlike the apparatus of the first embodiment in which the number of sensors 26 is greater than the number of pedals 20, the apparatus of the second embodiment includes fewer sensors 26 than the pedals 20.

An operation pedal unit 2c according to the second embodiment includes a plurality of pedals 20 operated when pressed downward so as to perform the function relating to the medical equipment, and a plurality of sensors 26 detecting the presence of the foot operating the pedals 20. The foot operating at least one of the plurality of pedals 20 is detected using the plurality of sensors 26. That is, when the control unit 61 receives detection information from the plurality of sensors 26 arranged adjacent to a particular one of the pedals 20 (a first pedal, i.e., the coagulation pedal 21 or the cutting pedal 22), the control unit 61 determines that the foot operating the particular pedal 20 (the first pedal, i.e., the coagulation pedal 21 or the cutting pedal 22) is present. In this manner, the presence of the foot approaching at least one of the pedals 20 can be detected using the plurality of sensors 26. Thus, the pedal 20 to which the foot of the operator O makes an approach can be identified with accuracy. This can avoid the decrease in the accuracy in detecting the presence of the foot operating the target pedal 20 among the plurality of pedals 20.

Specifically, six pedals 20 are provided. The pedals 20 include coagulation pedals 21, cutting pedals 22, a camera pedal 23, and a clutch pedal 24. The coagulation pedals 21 include coagulation pedals 21a and 21b. The cutting pedals 22 include cutting pedals 22a and 22b. In addition, five sensors 26 are provided. The sensors 26 include sensors 26a, 26b, 26c, 26d, and 26e.

The sensors 26a and 26b detect the foot approaching the coagulation pedal 21b. Specifically, if both of the sensors 26a and 26b detect the foot, the target pedal 20 is identified as the coagulation pedal 21b. The sensor 26c detects the foot approaching the cutting pedal 22b. Specifically, if the sensor 26c detects the foot, the target pedal 20 is identified as the cutting pedal 22b.

The sensor 26d detects the foot approaching the coagulation pedal 21a. Specifically, if the sensor 26d detects the foot, the target pedal 20 is identified as the coagulation pedal 21a. The sensor 26e detects the foot approaching the cutting pedal 22a. Specifically, if the sensor 26e detects the foot, the target pedal 20 is identified as the cutting pedal 22a.

The camera pedal 23 and the clutch pedal 24 are at a sufficient distance from the other pedals 20, and thus, no sensor 26 is provided for these pedals.

The other configurations of the second embodiment are the same as, or similar to, those of the first embodiment.

### [Third Embodiment]

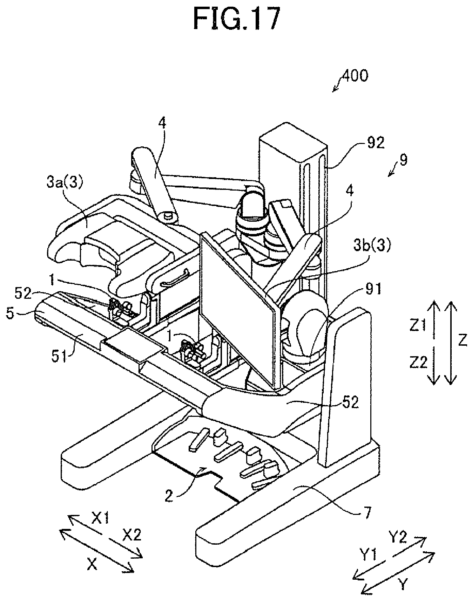

Next, referring to FIG. 17, a third embodiment of the present invention will be described below. Unlike the remote control apparatus of the first embodiment including a single display unit 3, the apparatus of the third embodiment includes a plurality of display units.

A remote control apparatus 400 of the third embodiment includes a plurality of display units 3 as shown in FIG. 17. In the example shown in FIG. 17, the remote control apparatus 400 is provided with both of the scope type display unit 3a and the non-scope type display unit 3b as the display units 3. The two display units 3 are arranged in the left and right directions (the X directions).

That is, the remote control apparatus 400 includes a plurality of (two) mounting counterparts 41. Specifically, the remote control apparatus 400 includes a plurality of (two) display unit supporting arms 4. The mounting counterparts 41 are respectively attached to the tip ends of the plurality of display unit supporting arms 4. Both of the scope type display unit 3a and the non-scope type display unit 3b can thus be attached to the remote control apparatus 400. This configuration can effectively improve the flexibility in terms of the display unit 3.

According to the third embodiment, one of the display units, namely, the non-scope type display unit 3b, shows at least one of the image of a surgical site previously taken, information about the state of the surgery, or operation information. For example, the non-scope type display unit 3b shows an X-ray image or a magnetic resonance image which is previously taken. The other one of the display units, namely, the scope type display unit or the non-scope type display unit, shows a 3D image taken by the endoscope 201b. In this manner, the operator O can carry out surgery while looking mainly at the endoscopic image on the other one of the display units during the surgery, and referring to at least one of auxiliary information items, such as the image of the surgical site previously taken, the information about the state of the surgery, or the operation information as needed, which can further improve the flexibility and extensibility of the remote control apparatus.

As can be seen in the foregoing, the scope type display unit 3a or the non-scope type display unit 3b as a main display unit 3 is selectively attachable to and detachable from the remote control apparatus 400 according to the third embodiment, and the non-scope type display unit 3b is attached as an auxiliary display unit. Thus, both of an immersive remote control apparatus and an open remote control apparatus are selectable, and the auxiliary information can also be referred to. The provision of the plurality of mounting counterparts makes it possible to freely select on which of the left side or the right side the main display unit is to be arranged.

In the example shown in FIG. 17, the scope type display unit 3a and the non-scope type display unit 3b are respectively attached to the two mounting counterparts 41. However, two scope type display units 3a may be attached to the two mounting counterparts 41, or two non-scope type display units 3b may be attached to the two mounting counterparts 41.

The other configurations of the third embodiment are the same as, or similar to, those of the first embodiment.

### [Fourth Embodiment]

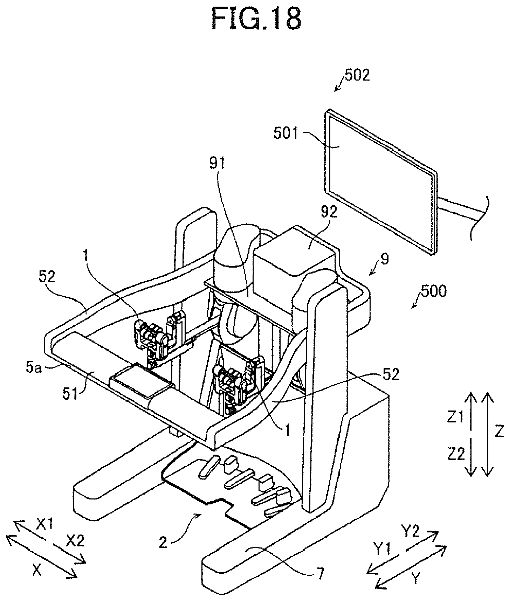

Next, referring to FIG. 18, a fourth embodiment of the present invention will be described below. Unlike the first and third embodiments in which the display unit is attached to the remote control apparatus, the fourth embodiment includes a display device provided independently of the remote control apparatus.

According to the fourth embodiment, a display device 501 is provided independently of a remote control apparatus 500 as shown in FIG. 18. That is, the remote control apparatus 500 has no display unit attached thereto. In addition, the remote control apparatus 500 has no display unit supporting arm for supporting the display unit. The remote control apparatus 500 and the externally provided display device 501 constitute a remote control system 502. This configuration of the remote control apparatus 500 can thus be simplified.

The display device 501 is arranged backward (toward the Y2 direction) of the remote control apparatus 500. Specifically, the display device 501 is arranged such that the operator O operating the remote control apparatus 500 can see the screen. The display device 501 may include a liquid crystal display, an organic EL display, or a plasma display, and show a 2D or 3D image taken by the endoscope 201b. The display device 501 may also show at least one of a surgical site image previously taken, information about the state of the surgery, or operation information. For example, the display device 501 may show an X-ray image or a magnetic resonance image which is previously taken.

The other configurations of the fourth embodiment are the same as, or similar to, those of the first embodiment.

### (Variations)

The embodiments disclosed herein are meant to be illustrative in all respects and should not be construed to be limiting in any manner. The scope of the present invention is defined not by the above-described embodiments, but by the scope of claims, and includes all modifications (variations) within equivalent meaning and scope to those of the claims.

For example, it has been described in the first to fourth embodiments that the pedals of the operation pedal unit include the coagulation pedals and the cutting pedals. However, this is not limiting. In the present invention, the pedals of the operation pedal unit may include such pedals, other than the coagulation pedals and the cutting pedals, which execute the function related to medical equipment.

Further, it has been described in the first to fourth embodiments that the sensors are blocking sensors. However, this is not limiting. In the present invention, the sensors may be through beam type sensors, or reflective type sensors. The presence of the foot may be detected without using light. For example, the detection may be performed with an ultrasonic wave or a coil. The sensors are not limited to non-contact sensors, and the presence of the foot may be detected via contact. For example, a mechanical switch may be used as the sensor.

It has been described in the first embodiment that the number of sensors is greater than the number of pedals, and in the second embodiment that the number of sensors is smaller than the number of pedals. However, this is not limiting. In the present invention, the number of sensors and the number of pedals may be the same. The number of sensors is not limited to five or six. The number of sensors may be four or less, or seven or more.

In the first to fourth embodiments, it has been described that one or two sensors are provided for a single pedal to detect the presence of the foot around the pedal. However, this is not limiting. In the present invention, three or more sensors may be provided for a single pedal to detect the presence of the foot around the pedal.

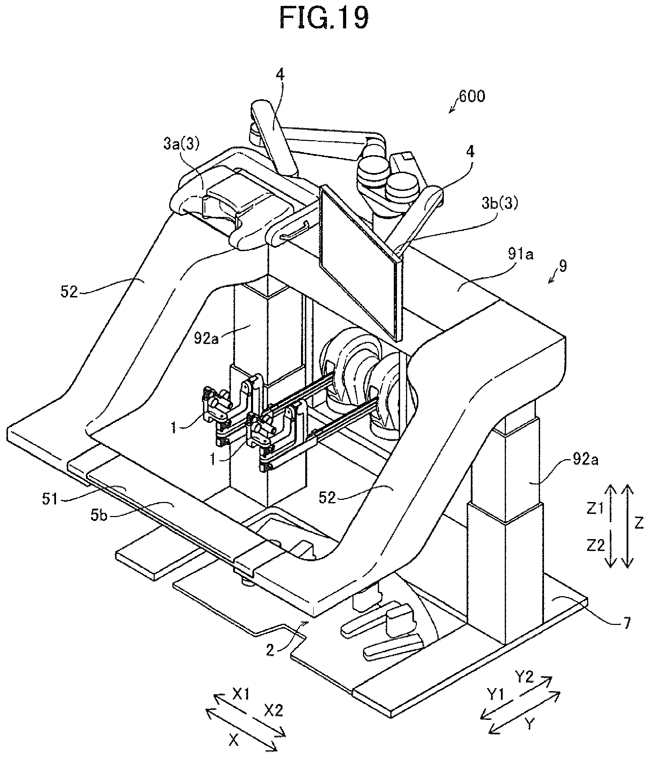

It has been described in the first and third embodiments that the connectors 52 of the arm rest 5 extend upward as they go forward (toward the operator O, or the Y1 direction). Further, it has been described in the fourth embodiment that the connectors 52 of the arm rest 5a extend in the horizontal direction. However, this is not limiting. In the present invention, as shown in FIG. 19 as a variation, the connectors 52 of the arm rest 5b may extend downward as they go forward. This configuration can save a wide space around the foot of the operator O.

Moreover, it has been described in the first to fourth embodiments that a single support mechanism 9 which moves the operating handle 1 and the arm rest 5 in the vertical direction is provided substantially at the center of the remote control apparatus in the left and right directions (the X directions). However, this is not limiting. According to the present invention, as shown in FIG. 19 as a variation, support mechanisms 9a may be arranged on the sides, in the left and right directions (the X directions), of a remote control apparatus 600. For example, the support mechanism 9a may include a support 91a and a pair of driving sections 92a. The support 91a may be supported by the pair of driving sections 92a arranged on the left and right sides of the remote control apparatus. The pair of driving sections 92a telescope in synchronization, thereby moving the support 91a up and down.

Further, it has been described in the first to fourth embodiments that the operation pedal unit is provided with the six pedals which are operated when pressed downward. However, this is not limiting. In the present invention, the operation pedal unit may include a plurality of pedals other than six pedals.

It has been described in the first embodiment that a single mounting counterpart 41, to which the display unit 3 is attached, is provided, and in the third embodiment that two mounting counterparts 41, to each of which the display units 3 is attached, are provided. However, this is not limiting. In the present invention, three or more mounting counterparts 41, to each of which the display unit 3 is attached, may be provided.

It has been described in the first to fourth embodiments that the attached display unit 3 is connected via cables to the remote control apparatus to be able to exchange information with the remote control apparatus. However, this is not limiting. In the present invention, the attached display unit 3 may be connected wirelessly to the remote control apparatus to be able to exchange information with the remote control apparatus.

Furthermore, it has been described in the first to fourth embodiments that the support mechanism moves the operating handle and the arm rest in the vertical direction. However, this is not limiting. In the present invention, the support mechanism may be configured to move the operating handle and the arm rest in the vertical direction and the horizontal direction as well.

## Claims

1. A remote control apparatus, comprising:
an operating handle (1) for remotely operating medical equipment (201a, 201b);
an operation pedal unit (2) including a plurality of pedals (20) each of which is operated when pressed downward by a foot to perform a function related to the medical equipment (201a, 201b), and a sensor (26) detecting the presence of the foot operating the pedals (20); and
a control unit (61) which receives detection information from the sensor (26), wherein
the sensor (26) includes a plurality of sensors (26) arranged near a first pedal (21, 22) among the plurality of pedals (20), and
when the control unit (61) receives the detection information from the plurality of sensors (26) arranged near the first pedal (21, 22), the control unit determines that the foot operating the first pedal (21, 22) is present.

2. The remote control apparatus of claim 1, wherein
the plurality of pedals (20) are arranged in a width direction (X) of the operation pedal unit (2).

3. The remote control apparatus of claim 2, wherein
at least one of the plurality of sensors (26) is arranged between the first pedal (21, 22) and a pedal adjacent to the first pedal (21, 22), and
the control unit (61) uses the detection information sent from the sensor (26) arranged between the first pedal (21, 22) and the pedal adjacent to the first pedal (21, 22) in order to determine whether the foot operating the first pedal (21, 22) is present or not, and whether the foot operating the pedal adjacent to the first pedal (21, 22) is present or not.

4. The remote control apparatus of claim 2 or 3, wherein
at least two of the plurality of sensors (26) arranged near the first pedal (21, 22) are disposed on both sides of the first pedal (21, 22) in the width direction (X).

5. The remote control apparatus of any one of claims 1 to 4, wherein
the operation pedal unit (2) further includes a base portion (2a) on which the plurality of pedals (20) are arranged, and a wall portion (2b) standing upright from the base portion (2a),
the sensor (26) includes a light emitter (261) and a light receiver (262), and
one of the light emitter (261) and the light receiver (262) is provided at the base portion (2a), and the other one of the light emitter (261) and the light receiver (262) is provided at the wall portion (2b).

6. The remote control apparatus of any one of claims 1 to 5, wherein
the plurality of pedals (20) include a coagulation pedal (21) which performs an operation of coagulating a surgical site, and a cutting pedal (22) which performs an operation of cutting the surgical site, and
the first pedal (21, 22) is the coagulation pedal (21) or the cutting pedal (22).

7. The remote control apparatus of claim 6, wherein
the plurality of pedals (20) include a plurality of coagulation pedals (21) and a plurality of cutting pedals (22), and
the plurality of coagulation pedals (21) and the plurality of cutting pedals (22) are alternately arranged in a width direction (X) of the operation pedal unit (2).

8. The remote control apparatus of claim 7, wherein
the plurality of coagulation pedals (21) and the plurality of cutting pedals (22) are alternately arranged on one side of the operation pedal unit (2) with respect to a center of the operation pedal unit in the width direction (X).

9. The remote control apparatus of claim 7 or 8, wherein
the coagulation pedals (21) include two coagulation pedals (21), and the cutting pedals (22) include two cutting pedals (22),
the two coagulation pedals (21) and the two cutting pedals (22) are the first pedals (21, 22), and
the number of sensors (26) that detect the presence of the foot operating the two coagulation pedals (21) and the two cutting pedals (22) is five.

10. The remote control apparatus of any one of claims 6 to 9, wherein
a top end of one of the coagulation pedal (21) and the cutting pedal (22) is located at a first vertical position, and a top end of the other one of the coagulation pedal (21) and the cutting pedal (22) is located at a second vertical position different from the first vertical position.

11. The remote control apparatus of any one of claims 1 to 10, wherein
the sensor (26) includes a single sensor (26) arranged near a second pedal (23, 24), which is different from the first pedal (21, 22), among the plurality of pedals (20), and
when the control unit (61) receives detection information from the single sensor (26) arranged near the second pedal (23, 24), the control unit determines that the foot operating the second pedal (23, 24) is present.

12. The remote control apparatus of claim 11, wherein
the plurality of pedals (20) are arranged in a width direction (X) of the operation pedal unit (2), and
a distance between the first pedal (21, 22) and a pedal adjacent to the first pedal (21, 22) is smaller than a distance between the second pedal (23, 24) and a pedal adjacent to the second pedal (23, 24).

13. The remote control apparatus of claim 12, wherein
the distance between the first pedal (21, 22) and the pedal adjacent to the first pedal (21, 22) is less than 40 mm.

14. The remote control apparatus of any one of claims 11 to 13, wherein
the plurality of pedals (20) includes a camera pedal (23) for operating an imaging unit which takes an image of a surgical site, and a clutch pedal (24) which temporarily blocks connection for operation between the medical equipment (201a, 201b) and the operating handle (1), and
the second pedal (23, 24) is the camera pedal (23) or the clutch pedal (24).

15. A method for identifying a target pedal to be operated among a plurality of pedals (20) each of which is operated when pressed downward by a foot to perform a function related to medical equipment (201a, 201b), the method comprising:
determining whether a plurality of sensors (26) arranged near any one of the pedals (20) has detected the foot operating the pedal (20); and
identifying, if the plurality of sensors (26) has detected the presence of the foot operating the pedal (20), the pedal (20) near the plurality of sensors (26) as the target pedal.
